# EUROPEAN PATENT SPECIFICATION

(11) **EP 3 791 789 B1**
(45) Date of publication and mention of the grant of the patent: **24.05.2023**
(21) Application number: 19774765.2
(22) Date of filing: 28.03.2019
(51) Int. Cl.: B65C 3/12, G01N 35/04

(54) **BLOOD-COLLECTING TUBE AUTOMATIC PREPARATION DEVICE**
AUTOMATISCHE VORBEREITUNGSVORRICHTUNG FÜR BLUTENTNAHMERÖHRCHEN
DISPOSITIF DE PRÉPARATION AUTOMATIQUE DE TUBE DE COLLECTE DE SANG

(30) Priority: 30.03.2018 JP 2018069376
(43) Date of publication of application: 17.03.2021
(73) Proprietor: Techno Medica Co., Ltd., Yokohama-shi, Kanagawa 224-0041 (JP); Fujitsu Frontech Limited, Inagi-shi, Tokyo 206-8555 (JP)
(72) Inventor: MATSUMOTO, Toshikazu, Yokohama-shi, Kanagawa 224-0041 (JP); SUGIMOTO, Koichi, Yokohama-shi, Kanagawa 224-0041 (JP); SHIRAISHI, Noriyuki, Yokohama-shi, Kanagawa 224-0041 (JP); CHONABAYASHI, Koji, Inagi-shi, Tokyo 206-8555 (JP); KOMAKI, Yasushi, Inagi-shi, Tokyo 206-8555 (JP); TSURU, Takamasa, Inagi-shi, Tokyo 206-8555 (JP)
(74) Representative: Mannucci, Michele
(86) International application number: PCT/JP2019/013662
(87) International publication number: WO 2019/189604

(56) References cited:
- JP-A- 2013 011 600
- JP-A- 2013 011 600
- JP-A- 2017 110 943
- JP-A- 2018 033 674
- JP-A- 2018 033 675
- JP-A- 2018 034 835
- JP-A- 2018 034 837
- JP-B2- 4 362 334
- JP-B2- 6 172 863
- US-B2- 9 505 563

## Description

### [Technical field]

This invention relates to an improved automatic blood sampling tube preparation device.

### [State of the art]

In a facility such as a hospital, blood is collected from a patient into a plurality of blood sampling tubes for examination.

In order to automatically and efficiently prepare the blood sampling tubes used for blood sampling, the applicant has already proposed an automatic blood sampling tube preparation device in which the device comprises
a plurality of blood-sampling tube containing units arranged one above the other,
a blood-sampling tube taking-out mechanism provided on each of the blood-sampling tube containing units, and
a label printing and pasting unit for printing the information about a patient on a label and pasting it on a blood-sampling tube taken out from the blood-sampling tube containing unit, which is arranged above the blood-sampling tube containing units,
wherein the preparation device is configured to collect the blood collection tube after labeling with a tray for each patient (See Patent Document 1).

The automatic blood-sampling tube preparation device proposed by the applicant has come to be used in many hospitals or the like, and recently it has been desired to downsize the preparation device so that it can be used in small hospitals and the like.

### [Prior Art Documents]

### [Patent Document]

[Patent document 1] Japanese patent No. 4362334
[Patent document 2] Japanese patent No. 6172863

### [Summary of the invention]

### [Problem to be solved by the invention]

The applicant has proposed an improved automatic blood-sampling tube preparation device for realizing the downsizing of the preparation device. In the improved automatic blood-sampling tube preparation device, instead of arranging a plurality of blood-sampling tube containing units vertically, they are arranged side by side in the front and rear, a transfer unit is arranged along the blood-sampling tube containing units under the containing units, a label printing and pasting unit is arranged below the transfer unit, and the label pasted blood-sampling tube is taken out from the containing units (See Patent Document 2).

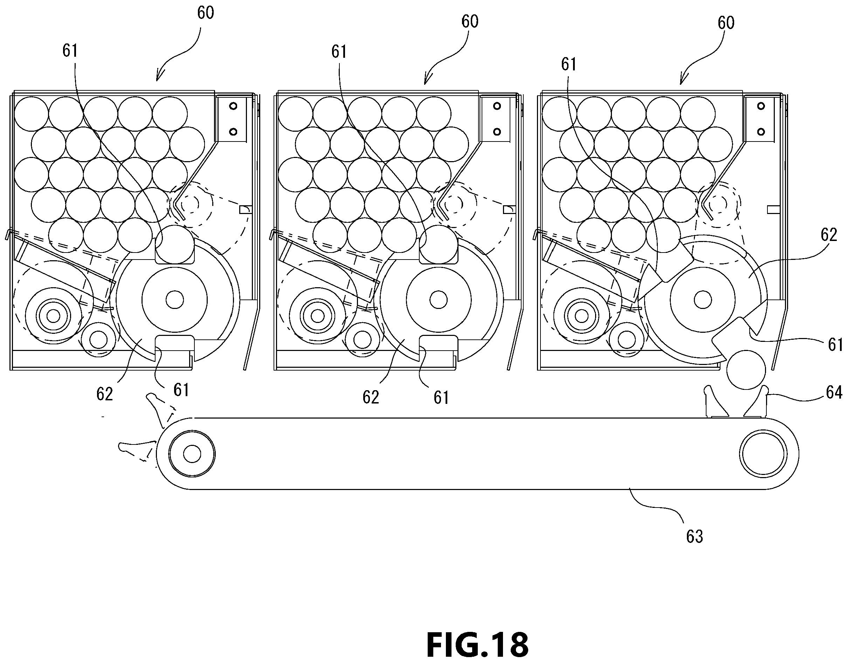

In the automatic blood-sampling tube preparation device proposed by the applicant in Patent Document 2 described above, as shown in FIG. 18, a taking-out roller 62 is provided at one corner of a lower portion of the blood-sampling tube containing unit 60, in which the roller 62 has a groove 61 capable of containing one blood-sampling tube. Said roller 62 is rotated in the clockwise direction in FIG. 18 so that the blood-sampling tube enters the groove 61. The roller 62 is further rotated in the clockwise direction so that the blood-sampling tube contained in the groove 61 is dropped and supplied to a bucket 64 of a transfer conveyer 63 arranged below the blood-sampling tube containing unit 60.

The applicant has continued to earnestly research and develop the automatic blood-sampling tube preparation device even after the above-mentioned preparation device was proposed for the purpose of further increasing the processing speed and downsizing of the automatic blood-sampling tube preparation device. As a result, the applicant has invented an automatic blood-sampling tube preparation device comprising a blood-sampling tube containing unit provided with a blood-sampling tube taking-out mechanism capable of further increasing the processing speed and reducing the size.

### [Means for solving the problem]

An automatic blood-sampling tube preparation device according to the present invention, comprising
a plurality of blood-sampling tube containing units for containing the blood-sampling tubes, which are arranged in a horizontal direction,
a transfer unit arranged under the blood-sampling tube containing units and along them,
a label printing and pasting unit for printing information about a patient on a label and pasting the printed label on a tube, which is arranged under the transfer unit, and
a blood-sampling tube collection unit for collecting label pasted tubes, which is arranged under the label printing and pasting unit,
characterized in that
each blood-sampling tube containing unit comprises a casing with an open bottom, capable of stacking and containing a plurality of blood-sampling tubes in a horizontal state in a direction orthogonal to the direction in which the blood-sampling tube containing units are arranged in parallel,
a blood-sampling tube taking-out cam is arranged at the center of the open bottom of each casing, which is configured to close the open bottom of the casing, to take-out the blood-sampling tube contained in the casing one by one, and to transfer the taken out tube to the transfer unit arranged below the casing,
said blood-sampling tube taking-out cam is configured to extend along a longitudinal direction of the blood-sampling tube containing unit and to be rotatable left and right about a longitudinal axis thereof,
a convex portion is formed on an upper portion of the blood-sampling tube taking-out cam, in which said convex portion has a mountain-shaped cross section protruding upward, and forms symmetrical inclined surfaces extending downwardly from a top thereof to side walls of the blood-sampling tube containing unit said inclined surfaces extending along the longitudinal direction of the convex portion,
a pair of left and right grooves extending along the longitudinal direction of the blood-sampling tube taking-out cam at the lower part thereof,
said taking-out cam being configured to direct said convex portion to the inside of the blood-sampling tube containing unit to form the bottom surface of the blood-sampling tube containing unit and to direct each groove thereof to +the corresponding side wall when the taking-out cam is at its center position,
wherein
when the blood-sampling tube taking-out cam is rotated in one direction form its center position, one of grooves appears inside the blood-sampling tube containing unit so that one of blood-sampling tubes contained in the blood-sampling tube containing unit enters one groove while the blood-sampling tube contained in other groove is dropped and supplied to the transfer unit,
and when the blood-sampling tube taking-out cam is rotated in other direction form its center position, said other groove appears inside the blood-sampling tube containing unit so that one of blood-sampling tubes contained in the blood-sampling tube containing unit enters said other groove while the blood-sampling tube contained in said one groove is dropped and supplied to the transfer unit.

Further, inclined portions may be provided at portions of both walls of the blood-sampling tube containing unit, corresponding to the inclined surfaces of the convex portion, each of the inclined portions is inclined obliquely downward toward the lower end of each inclined surface.

Preferably, the convex portion of the blood-sampling tube taking-out cam may be shaped in its cross section so that its profile defining the inclined surfaces is within a true circle having a radius extending from a rotational axis of the blood-sampling tube taking-out cam to the top of the convex portion.

In addition, one end portion in the longitudinal direction of the blood-sampling tube taking-out cam may be formed in a stepped shape so as to avoid a cap portion of the blood-sampling tube from interfering with the blood-sampling tube taking-out cam.

Further, the blood-sampling tube containing unit may be configured to be capable adjusting its length in the longitudinal direction according to the length of the blood-sampling tube to be contained.

Furthermore, the blood-sampling tube containing unit may be configured to be capable adjusting its width according to the number of the blood-sampling tubes to be contained.

Further, protrusions may be provided at portions of both walls of the blood-sampling tube containing unit, corresponding to the top of the convex portion or above the top of the convex portion, said protrusions are extending in longitudinal direction of the convex portion.

Furthermore, a continuous protrusion extending the longitudinal direction or a plurality of protrusions intermittently arranged along the longitudinal direction may be formed on the top of the convex portion of the blood-sampling tube taking-out cam.

Alternatively, a continuous protrusion extending in the longitudinal direction or a plurality of protrusions intermittently arranged along the longitudinal direction may be formed on the inclined surfaces formed by the convex portion of the blood-sampling tube taking-out cam.

In addition, each of the inclined surfaces formed on the convex portion of the blood-sampling tube taking-out cam may have a stepped shape.

### [Effect of the invention]

An automatic blood-sampling tube preparation device according to the present invention, comprising
a plurality of blood-sampling tube containing units for containing the blood-sampling tubes, which are arranged in a horizontal direction,
a transfer unit arranged under the blood-sampling tube containing units and along them,
a label printing and pasting unit for printing information about a patient on a label and pasting the printed label on a tube, which is arranged under the transfer unit, and
a blood-sampling tube collection unit for collecting label pasted tubes, which is arranged under the label printing and pasting unit,
characterized in that
each blood-sampling tube containing unit comprises a casing with an open bottom, capable of stacking and containing a plurality of blood-sampling tubes in a horizontal state in a direction orthogonal to the direction in which the blood-sampling tube containing units are arranged in parallel,
a blood-sampling tube taking-out cam is arranged at the center of the open bottom of each casing, which is configured to close the open bottom of the casing, to take-out the blood-sampling tube contained in the casing one by one, and to transfer the taken out tube to the transfer unit arranged below the casing,
said blood-sampling tube taking-out cam is configured to extend along a longitudinal direction of the blood-sampling tube containing unit and to be rotatable left and right about a longitudinal axis thereof,
a convex portion is formed on an upper portion of the blood-sampling tube taking-out cam, in which said convex portion has a mountain-shaped cross section protruding upward, and forms symmetrical inclined surfaces extending downwardly from a top thereof to side walls of the blood-sampling tube containing unit said inclined surfaces extending along the longitudinal direction of the convex portion,
a pair of left and right grooves extending along the longitudinal direction of the blood-sampling tube taking-out cam at the lower part thereof,
said taking-out cam being configured to direct said convex portion to the inside of the blood-sampling tube containing unit to form the bottom surface of the blood-sampling tube containing unit and to direct each groove thereof to the corresponding side wall when the taking-out cam is at its center position,
wherein
when the blood-sampling tube taking-out cam is rotated in one direction form its center position, one of grooves appears inside the blood-sampling tube containing unit so that one of blood-sampling tubes contained in the blood-sampling tube containing unit enters one groove while the blood-sampling tube contained in other groove is dropped and supplied to the transfer unit,
and when the blood-sampling tube taking-out cam is rotated in other direction form its center position, said other groove appears inside the blood-sampling tube containing unit so that one of blood-sampling tubes contained in the blood-sampling tube containing unit enters said other groove while the blood-sampling tube contained in said one groove is dropped and supplied to the transfer unit.

As described above, the blood-sampling tube taking-out cam is configured to be not rotated in one direction but to be rotated left and right so that every time the blood-sampling tube taking-out cam is rotated left or right, the contained blood-sampling tube is dropped and supply from one groove while the blood-sampling tube is contained in the other groove. As a result, it is possible to always contain the blood-sampling tube in one of the grooves and wait for supplying the blood-sampling tube, and the rotation amount of the blood-sampling tube taking-out cam may be significantly reduced as compared with the case of rotating in one direction. Therefore, according to the present invention, it is possible to increase the taking-out speed of the blood-sampling tube. Furthermore, by reducing the amount of rotation of the blood-sampling tube taking-out cam, it is possible to avoid biting due to competition with other blood-sampling tubes even if a smaller-diameter tube runs wild in the groove of the cam. As a result, it is possible to take out the smaller-diameter tube and a larger-diameter tube with the same blood-sampling tube taking-out cam. Further, since the convex portion is formed on the upper portion of the blood-sampling tube taking-out cam, in which said convex portion has the mountain-shaped cross section protruding upward, and forms symmetrical inclined surfaces extending downwardly from the top thereof to right and side walls of the blood-sampling tube containing unit, and said inclined surfaces extend along the longitudinal direction of the convex portion, it is possible to make the inclined bottom surfaces which incline downwardly in left and right direction from the center thereof. As a result, it is possible to distribute the blood-sampling tubes contained in the blood-sampling tube containing unit, to left and right at the bottom surface thereof. Therefore, it is possible to take out the blood-sampling tubes contained in the blood-sampling tube containing unit without bias.

Furthermore, since inclined portions are provided at portions of both walls of the blood-sampling tube containing unit, corresponding to the inclined surfaces of the convex portion, each of the inclined portions is inclined obliquely downward toward the lower end of each inclined surface, it is possible to form V-shaped spaces between each inclined surface of the convex portion and the inclined portions of the both side walls and hold a blood-sampling tube to be contained in the groove next in each of the V-shaped spaces.

In addition, since the convex portion of the blood-sampling tube taking-out cam is shaped in its cross section so that its profile defining the inclined surfaces is within the true circle having the radius extending from the rotational axis of the blood-sampling tube taking-out cam to the top of the convex portion, the inclination angle of the inclined surfaces formed by the convex portion becomes a relatively acute angle. As a result, when the blood-sampling tube taking-out cam is rotated, it is easier to distribute the blood-sampling tubes contained in the blood-sampling tube containing unit to the left and right sides of the convex portion.

Furthermore, since the protrusions are provided at portions of both walls of the blood-sampling tube containing unit, corresponding to the top of the convex portion or above the top of the convex portion, and said protrusions are extending in longitudinal direction of the convex portion, it is possible to limit the number of blood-sampling tubes to be entered between each inclined surface and each side wall. As a result, it is possible to reduce the biting of the blood-sampling tubes.

Further, since the continuous protrusion extending the longitudinal direction or the plurality of protrusions intermittently arranged along the longitudinal direction are formed on the top of the convex portion of the blood-sampling tube taking-out cam and also the convex portion is formed in the shape of the mountain, every time the blood-sampling tube taking-out cam is rotated, the blood-sampling tubes contained in the blood-sampling tube containing unit may be pushed up and moved to align the tubes. As a result, the problems that the attitude of the blood-sampling tube is inclined and the blood-sampling tube can not enter the groove or the like does not occur.

The same effect as described above may be obtained by the configuration in that a continuous protrusion extending the longitudinal direction or a plurality of protrusions intermittently arranged along the longitudinal direction are formed on the inclined surface formed by the convex portion of the blood-sampling tube taking-out cam.

Furthermore, since each of the inclined surfaces formed on the convex portion of the blood-sampling tube taking-out cam is formed into the stepped shape, the lower end (that is, the portion opposite to the cap) of the blood-sampling tube located near the blood-sampling tube taking-out cam falls to the stepped portion so that the direction of the blood-sampling tube is aligned with the longitudinal direction of the blood-sampling tube taking-out cam. This configuration prevents the attitude of the blood-sampling tube from tilting obliquely with respect to the longitudinal direction of the blood-sampling tube taking-out cam during it is rotated.

### [Brief description of drawings]

Fig. 1 is an external view of an automatic blood-sampling tube preparation device.
FIG. 2 is a right side view of the preparation device shown in FIG. 1 in which a right side panel of a housing is omitted.
FIG.3 is a schematic front view of a blood-sampling tube containing unit 1.
FIG. 4 is a schematic enlarged view of the periphery of a blood-sampling tube taking-out cam 13 of the blood-sampling tube containing unit 1 shown in FIG.3.
FIG. 5 is a schematic top view of the blood-sampling tube containing unit 1 shown in FIG. 4.
Fig. 6 is a schematic cross-sectional view taken along line A-A in Fig. 5.
FIG. 7(a) is a schematic top view of the blood-sampling tube taking-out cam 13 and FIG. 7(b) is a cross-sectional view taken along the line B-B in FIG. 7(a).
FIG. 8(a) is an enlarged view around the blood-sampling tube taking off cam in the blood sampling-tube containing unit 1 in which sensors are shown, and FIG. 8(b) is a view showing a state where the sensors are separated from the blood-sampling tube containing unit 1.
FIG.9 (a) to (g) are diagrams showing a process of taking out blood-sampling tubes by the blood-sampling tube taking-out cam 13 from the blood-sampling tube containing unit 1.
Fig. 10 is a diagram showing the operation of a transport bucket.
FIG. 11(a) shows a state in which a pressure roller 37 is in a blood-sampling tube receiving position, FIG. 11(b) shows a state in which the pressure roller 37 is in a label pasting position, and FIG. 11(c) shows a state in which the pressure roller 37 is in the blood-sampling tube discharge position.
FIG. 12 shows a side view of one of a plurality of blood-sampling tube containing unit 40 arranged horizontally in a second embodiment of an automatic blood-sampling tube preparation device according to the present invention.
Fig. 13 is a cross-sectional view taken along line A-A in Fig. 12.
Fig. 14 is a cross-sectional view taken along line B-B in Fig. 13.
FIG. 15 is a schematic enlarged view of a portion of a circle X in FIG. 13.
FIG. 16 shows a perspective view of a part of a housing and a cam.
FIG. 17 is a perspective view of a third embodiment of an automatic blood-sampling tube preparation device according to the present invention corresponding to FIG. 16.
FIG. 18 is a view showing blood-sampling tube containing units and blood-sampling tube taking off mechanism in a conventional automatic blood-sampling tube preparation device.

### [MODE FOR CARRYING OUT THE INVENTION]

Hereinafter, an automatic blood-sampling tube preparation device according to the present invention will be described with reference to embodiments shown in the accompanying drawings.

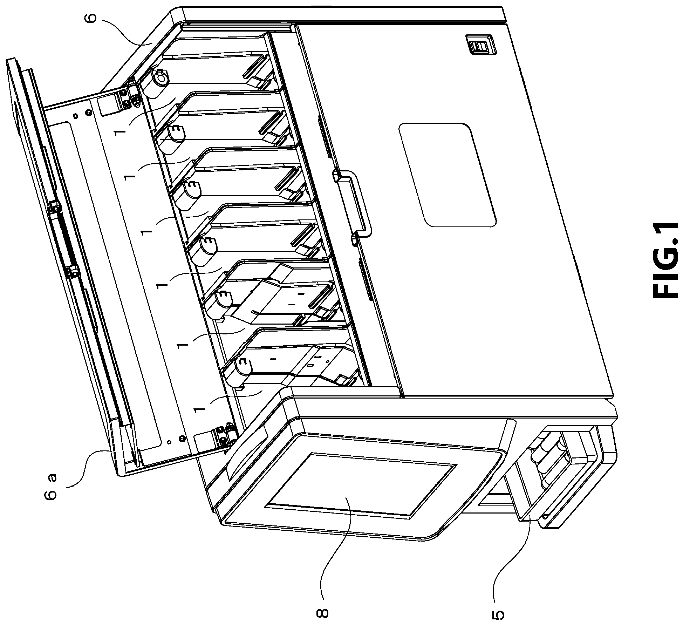

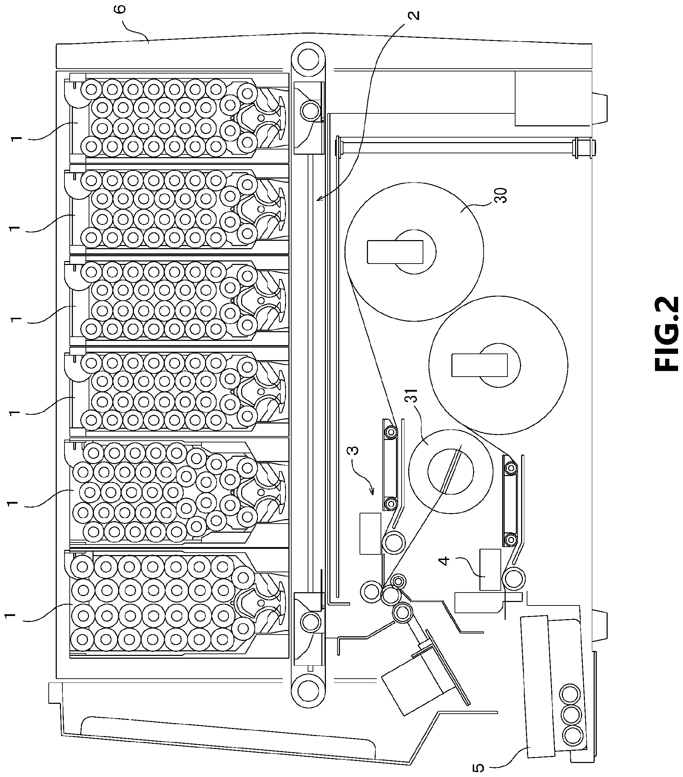

First of all, a layout of each component of the automatic blood-sampling tube preparation device will be described with reference to FIGS. 1 and 2.

Fig. 1 is an external view of an automatic blood-sampling tube preparation device, and FIG. 2 is a right side view of the preparation device shown in FIG.1 in which a right side panel of a housing is omitted.

As shown in the drawings, the automatic blood-sampling tube preparation device comprises:
six blood-sampling tube containing units 1 arranged horizontally in a line,
a transfer unit 2 arranged below the blood-sampling tube containing units 1,
a label printing and pasting unit 3 and a label printer 4 arranged further below the transfer unit 2, and
a tray 5 arranged so as to collect one or more blood-sampling tubes pasted with labels and a printed label for hand-pasting.

In the figure, reference numeral 6 indicates a housing that houses each of the above-described components. As shown in the drawings, the blood-sampling tube containing units 1, the transfer unit 2, the label printing and pasting unit 3 and the label printer for hand pasting 4 are accommodated inside the housing 6, and the tray 5 is externally provided so that a user can replace it.

According to the automatic blood-sampling tube preparation device configured as described above, a blood-sampling tubes is taken out from the blood-sampling tube containing unit 1 that contain the blood-sampling tubes necessary for the examination for a patient based on the instruction from the doctor. The blood-sampling tube taken out from the blood-sampling tube containing unit 1 is transferred to the transfer unit 2, and the transfer unit 2 transfers the received blood-sampling tube to the label printing and pasting unit 3.

On the other hand, the label printing and pasting unit 3 prints the information about the patient corresponding to the transferred blood-sampling tube on a label, pastes the printed label to the transferred blood-sampling tube, and discharges the label pasted tube to the tray 5.

At the same time, the label printer 4 for hand-pasting prints a blood sampling order instruction paper based on the blood sampling information from the doctor. If necessary (that is, in the case that a blood-sampling tube required to the examination of the patient is not contained in any blood sampling-tube containing units 1), the label printer 4 prints a label for hand-pasting. The blood-sampling order instruction paper and the label for hand-pasting are discharged to the tray 5.

The above-described operation is repeated until all blood-sampling tubes required to the examination of one patient are collected in the tray 5. When the all blood-sampling tubes for one patient are collected in the tray 5, some visual and/or sound signal indicating the finishing of the process is outputted so that the user replaces the tray 5 with a next new tray 5.

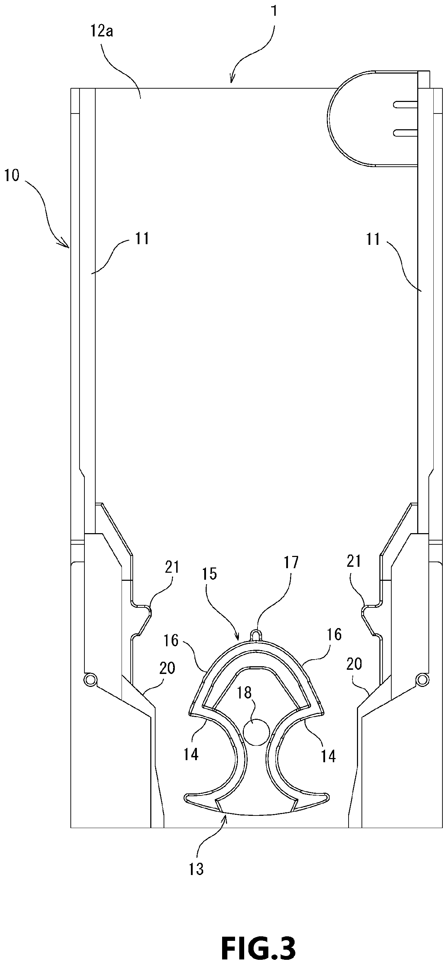

Hereinafter, the configuration of the blood-sampling tube containing unit 1 will be described with reference to FIG. 3. FIG.3 is a schematic front view of a blood-sampling tube containing unit 1.

It is note that, as shown in FIG. 2, the six blood-sampling tube containing units 1 are not all completely the same in configuration and the size and the configuration are slightly different depending on the thickness and the number of the blood-sampling tube to be contained. However, the main structure of all blood-sampling tube containing units 1 are substantially the same. Therefore, in the following description, the configuration of the leftmost blood-sampling tube containing unit 1 in FIG. 2 will be described.

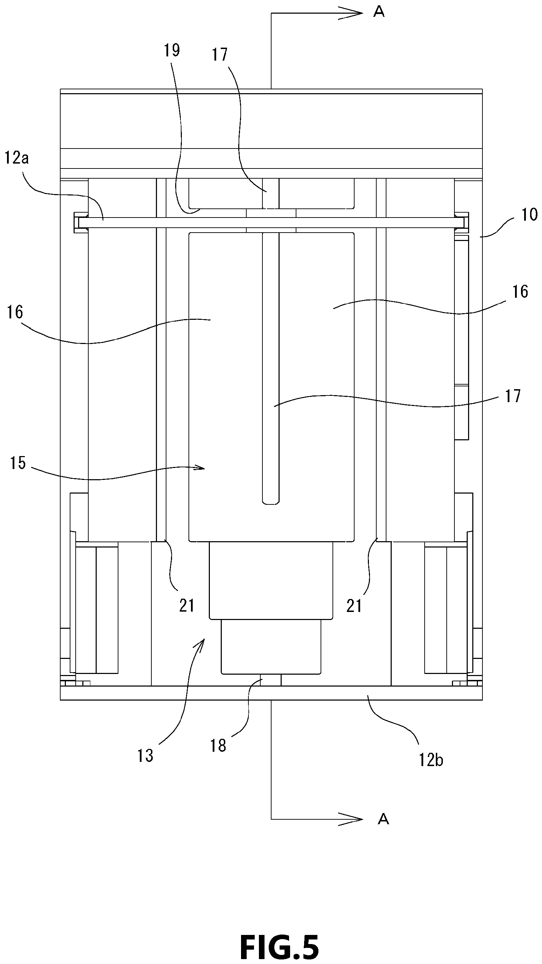

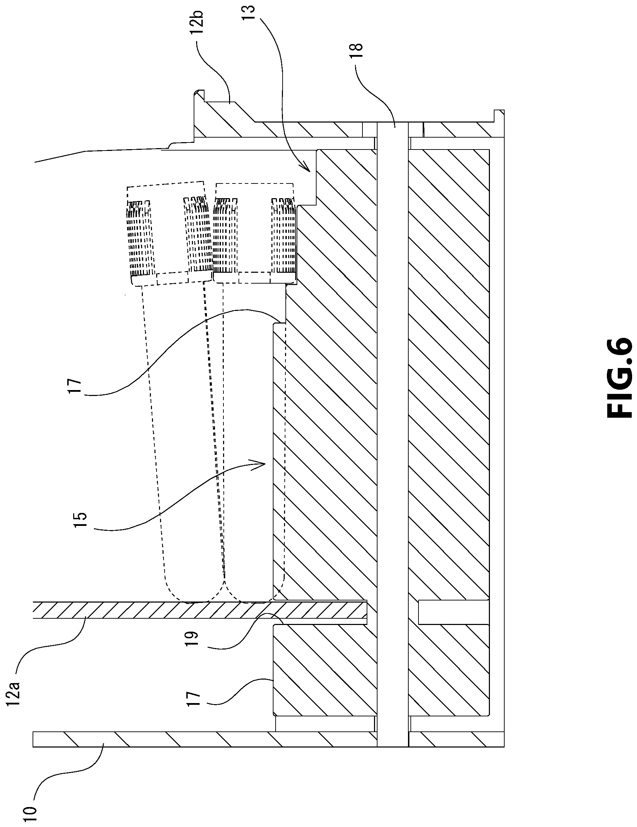

The blood-sampling tube containing unit 1 has a casing 10 capable of stacking and housing a plurality of blood-sampling tubes in a horizontal state. The casing 10 includes a pair of left and right side walls 11, a rear wall 12a, and a front wall 12b. The front wall 12b covers a lower portion of the front surface of the casing 10 (see FIGS. 5 and 6). A bottom surface, an upper surface, and an upper portion of the front surface of the casing 10 are opened. A blood-sampling tube taking-out cam 13 is provided in the opening portion of the bottom surface of the casing 10.

The blood-sampling tube taking-out cam 13 is configured to take out the blood collection tubes contained in the blood-blood sampling tube containing unit 1 one by one and drop the taken blood-sampling tube into the transfer unit 2 located below.

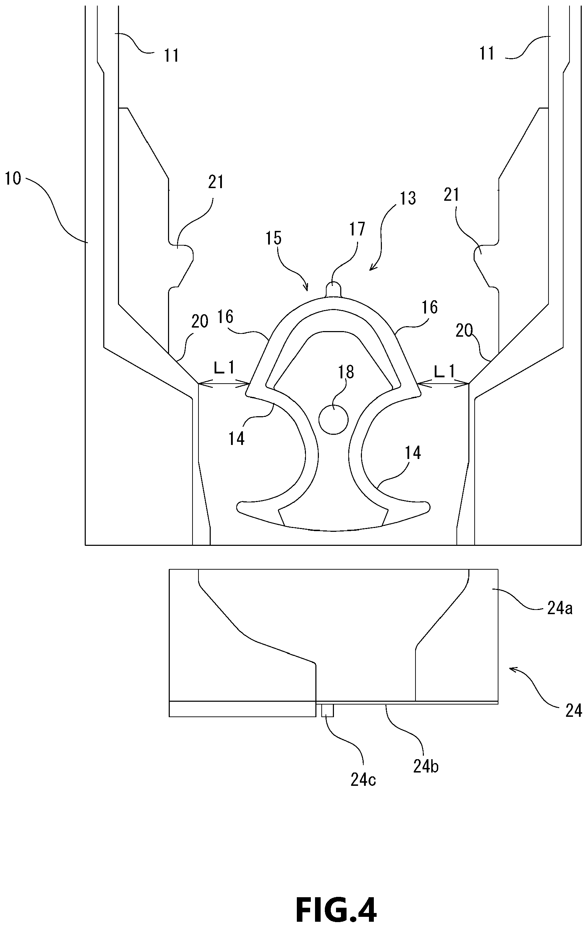

Hereinafter, the configuration of the blood-sampling tube taking-out cam 13 will be described with reference to FIGS. 4 to 7.

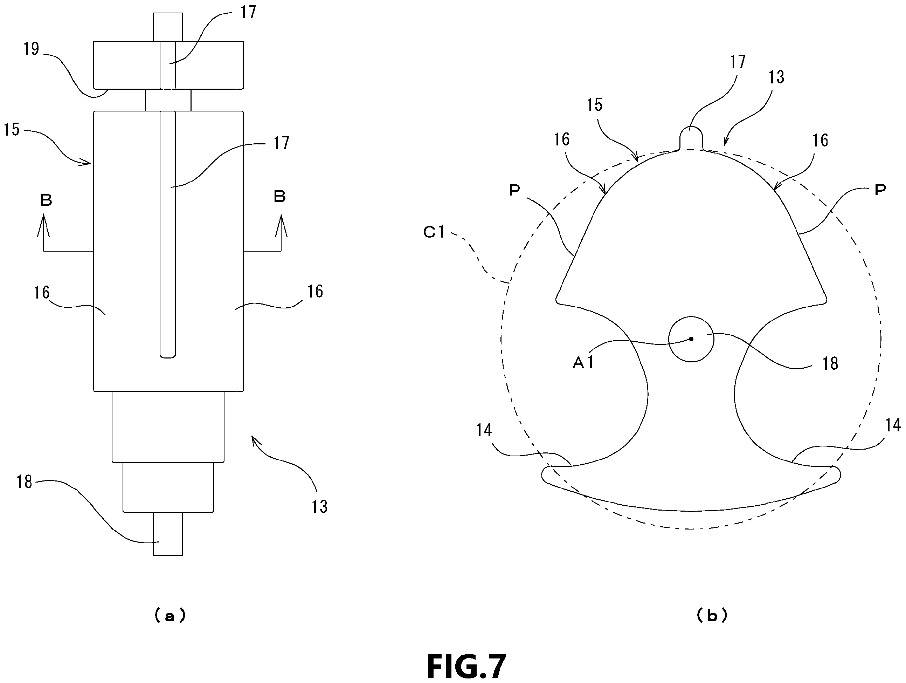

FIG. 4 is a schematic enlarged view of the periphery of a blood-sampling tube taking-out cam 13 of the blood-sampling tube containing unit 1 shown in FIG.3. FIG. 5 is a schematic top view of the blood-sampling tube containing unit 1 shown in FIG. 4. Fig. 6 is a schematic cross-sectional view taken along line A-A in Fig. 5. FIG. 7(a) is a schematic top view of the blood-sampling tube taking-out cam 13 and FIG. 7(b) is a cross-sectional view taken along the line B-B in FIG. 7(a).

As shown in FIGS. 5 and 6, the blood-sampling tube taking-out cam 13 has a length extending over the entire length of the casing 10 and is arranged in the widthwise center of the casing 10 so as to close the bottom opening of the casing 10.

Further, the blood-sampling tube taking-out cam 13 has a stepped shape has a reduced diameter at the front portion thereof so that a cap of the blood-sampling tube can be avoided from the cam 13 when the blood-sampling tubes are accommodated in the casing 10.

As shown in FIGS. 3 and 4, the blood-sampling tube taking-out cam 13 has a pair of left and right recessed grooves 14 extending in the longitudinal direction in the lower portion thereof. Each of the grooves 14 is dimensioned to accommodate one blood-sampling tube contained in the blood-sampling tube containing unit 1.

An upper part of the blood-sampling tube taking-out cam 13 is formed with a convex portion 15 which projects inward (that is, upward) of the blood-sampling tube containing unit 1, and the convex portion 15 forms a pair of bottom surfaces (inclined surfaces) 16 that are symmetrical each other when the blood-sampling tube taking-out cam 13 is at the center position thereof.

Specifically, in this embodiment, as shown in FIG.7(b), the convex portion 15 of the blood -sampling tube taking-out cam 13 is shaped in its cross-sectional shape so that its profile P defining the inclined surfaces 16 is within a true circle C having a radius extending from a rotational axis A1 of the blood-sampling tube taking-out cam 13 to an apex of the convex portion 15.

As a result, the convex portion 15 forms a relatively acute-angled inclined surfaces 16 centering on the apex of the blood-sampling tube taking-out cam 13 when the cam 13 is at the center position thereof.

A protrusion 17 extending along the longitudinal direction of the blood-sampling tube taking-out cam 13 is formed on the apex of the convex portion 15 of the blood - sampling tube taking-out cam 13.

As shown in FIG.1, several blood-sampling tubes are stacked and stored in the blood-sampling tube containing unit 1. As the blood-sampling tube is taken out from the blood-sampling tube containing unit 1, the blood-sampling tube group accommodated in the blood-sampling tube containing unit 1 are fall downward. As a result, the attitude of the blood-sampling tube(s) located on the blood-sampling tube group may be inclined. If the inclined blood-sampling tube is fall downward as it is, there is a possibility that the inclined tube cannot enter the groove 14 formed in the blood-sampling tube taking-out cam 13.

The protrusion 17 solves above problem, i.e. the protrusion 17 slightly moves the blood-sampling tube located on the blood-sampling tube group upward every time the blood-sampling tube taking-out cam 13 is rotated, so that the attitude of said blood-sampling tube is aligned by the movement thereof.

Said blood-sampling tube taking-out cam 13 is configured to be rotatable left and right about a rotation shaft 18 thereof by a motor or the like (not shown). Specifically, when the cam 13 is rotated a certain amount in the right direction, the blood-sampling tube enters the groove 14 located on the left side of the cam 13 whereas the blood-sampling tube accommodated in the groove 14 located on the right side of the cam 13 falls into the transfer unit 2. Conversely, when the cam 13 is rotated a certain amount to the left, the blood-sampling tube enters the groove 14 located on the right side of the cam 13 whereas the blood-sampling tube accommodated in the groove 14 located on the left side of the cam 13 falls into the transfer unit 2.

The amount of rotation of the cam 13 can be appropriately adjusted according to the size of the blood-sampling tube accommodated in the blood-sampling tube containing unit 1.

Further, as shown in FIGS. 5 to 7, the cam 13 has an annular notch 19 formed at the rear side in the longitudinal direction thereof. In this embodiment, the blood - sampling tube containing unit 1 is configured so that the length of the casing 10 can be adjusted according to the tube length of the blood-sampling tube to be used. When the rear wall 12a for adjusting the length of the casing 10 is attached the notch 19 prevents the rear wall 12a from coming into contact with the cam 13.

On both side walls 11 of the casing 10, as shown in FIG. 4, inclined portions 20 which are inclined obliquely downward in the direction toward the convex portion 15 of the cam 13 are formed. Before the cam 13 rotates and the blood-sampling tube enters the groove 14, the blood-sampling tube is held between the inclined portion 20 and the inclined surface 16 formed on the convex portion 15. That is, since a horizontal distance L1 (refer to FIG. 4) between the end of the inclined portion 20 and the inclined surface 16 is smaller than the diameter of the blood-sampling tube, the blood-sampling tube cannot enter the groove 14. By providing the inclined surfaces 20 on the side walls 11 as described above, a V-shaped space is formed between each inclined surface 16 and the corresponding inclined portion 20 especially when the cam 13 is at the center position. Then, one blood-sampling tube is guided to the lowermost end of the V-shaped space and held at that position.

Further, protrusions 21 extending in the longitudinal direction of the casing 10 are formed above the inclined portions 20 of both side walls 11. The projection 21 is arranged so as to located slightly above the top of the convex portion 15 when the cam 13 is at the center position thereof so that the plural blood-sampling tubes are prevented from entering between each inclined surface 16 of the convex portion 15 of the cam 13 and the corresponding inclined portions 20 formed on both side walls 11. As a result, only one blood-sampling tube is held between the inclined surfaces 16 of the convex portion 15 and each of the inclined portions 20 formed on the side walls 11 respectively so that the plural tubes are prevented from entering each space between the inclined surfaces 16 and each of the inclined portions 20 and from causing biting that hinders the rotation of the cam 13.

In particular, as will be clearly understood with reference to FIG. 6,m in this embodiment the rear wall 12a of the casing 10 is configured to be slidable upward and removable. By removing the rear wall 12a, the longitudinal distance of the blood-sampling tube containing unit 1 can be extended, and thus it becomes possible to contain blood-sampling tubes longer than the blood-sampling tubes used in this embodiment. The configuration of the rear wall 12a is not limited to this embodiment, but may be configured to move back and forth.

Furthermore, the wide of the casing 10 of the blood-sampling tube containing unit 1 may be set according to the number and the diameter of the blood-sampling tubes to be contained.

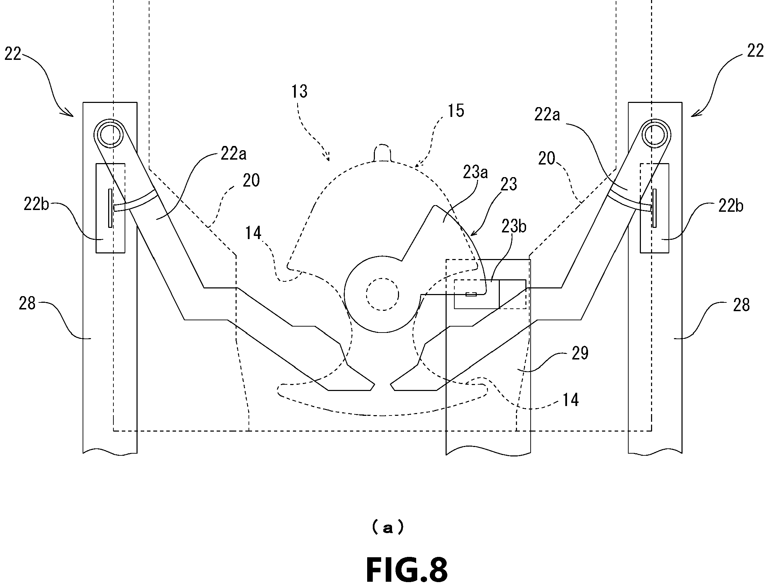

Next, the configuration of the blood-sampling tube detection sensor and cam origin position detection sensor will be described. FIG. 8A is an enlarged view of the vicinity of the blood-sampling tube taking-out cam in the blood-sampling tube containing unit 1 for explaining the configuration of these sensors.

A blood-sampling tube detection sensor 22 that detects whether the blood-sampling tube is accommodated in the groove 14 of the cam 13 is provided in the blood-sampling tube containing unit 1 as described above. The detection sensor 22 has a pair of arms 22a and sensors 22b. One end of each arm 22a enters each of the grooves 14 and the other end of each arm 22a is rotatably supported by each bracket 28 that is provided on the transfer unit 2 located under the blood-sampling tube containing unit 1 so as to extend upward. Each sensor 22b detects the movement of each arm 22a. Each arm 22a is configured to be pushed downward by the blood-sampling tube when said tube is contained in the corresponding groove 14 and to be returned to the corresponding groove 14 again when said tube is discharged from the groove 14. By detecting the movement of each arm 22a by the corresponding sensor 22b, whether the blood-sampling tube is contained in the corresponding groove 14 is detected. For example, each sensor 22b may be a light transmission type sensor, and by detecting the ON or OFF state of the light transmission type sensor, it is possible to detect whether or not the blood-sampling tube is accommodated in the corresponding groove 14.

A cam origin position detection sensor 23 has a substantially fan-shaped shield plate 23 and a sensor 23b. The shield plate 23 is rotated together with the cam 13 and the sensor 23b detects the position of the shield plate 23a when the cam 13 is at origin position thereof. For example, the sensor 23b may be a light transmission type sensor having an emitter and a receiver and the shield plate 23a may be arranged between the emitter and the receiver. In this case, while the shield plate 23a is rotated together with the cam 13 the shield plate 23a shields the sensor 23b so that the sensor 23b is switched from ON to OFF or from OFF to ON. Then it is possible to determine the origin position by detecting the switching timing of the sensor 23b. Said sensor 23b is fixed on a bracket 29 provided on the transfer unit 2 located under the blood-sampling tube containing unit 1 so as to extend upward.

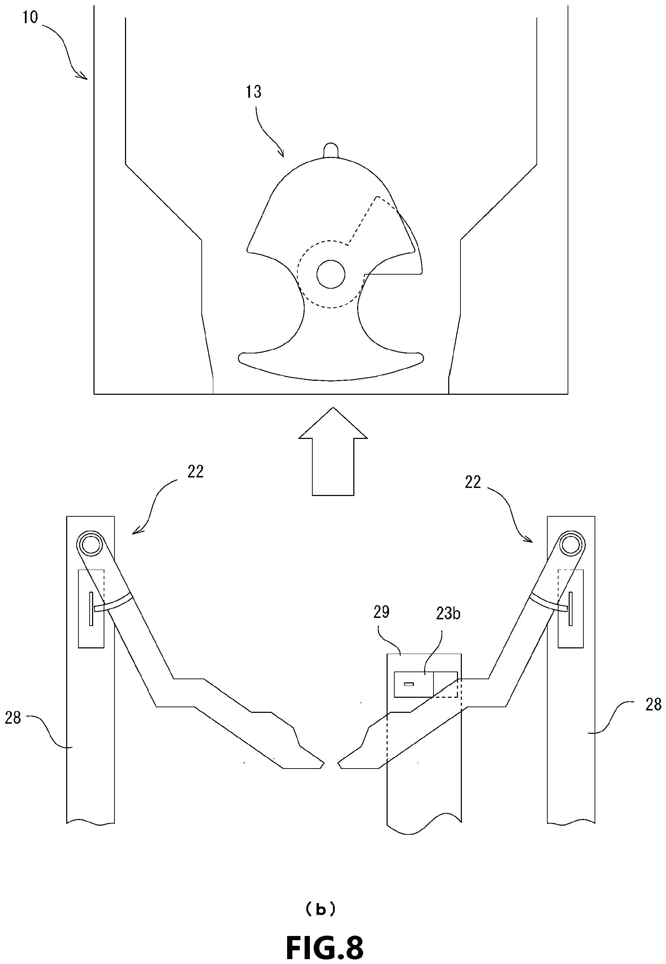

As described above, in this embodiment, since the blood-sampling tube detection sensors 22 are arranged on the brackets 28 provided on the transfer unit2, not on the casing 10 of the blood-sampling tube containing unit 1 the casing 10 can be removed by sliding it upward so that the casing 10 is separated from the sensors 22 (See FIG. 8(b)). As a result, the maintenance of the sensors 22 becomes easy.

Similarly, in this embodiment, since the sensor 23b of the cam origin position detection sensor 23 is arranged on the bracket 29 provided on the transfer unit 2, not on the casing 10 of the blood-sampling tube containing unit 1 the casing 10 can be removed by sliding it upward so that the casing 10 is separated from the sensor 23b (See FIG. 8(b)). As a result, the maintenance of the sensor 23 becomes easy.

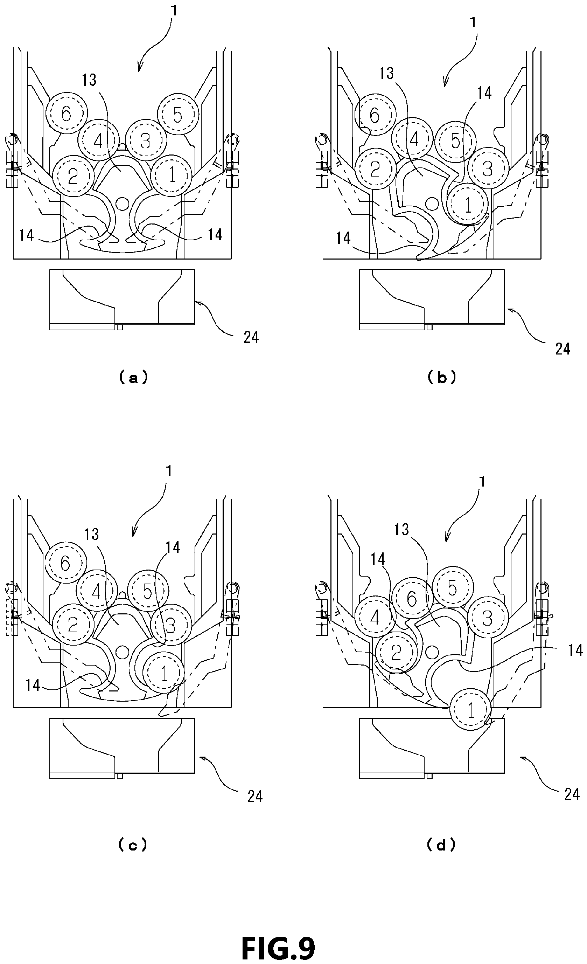

Hereinafter, a step of taking out the blood-sampling tube by the blood-sampling tube taking-out cam 13 in the blood-sampling tube containing unit 1 will be described with reference to FIGS. 9(a) to 9(g).

FIG. 9(a) shows a state in which the blood-sampling tube taking-out cam 13 is at the center position thereof. As shown in the drawing, when the cam 13 is at the center position thereof, the convex portion 15 faces inward of the blood-sampling tube containing unit 1 and forms a bottom wall that closes the bottom opening of the casing 10 with the inclined surfaces 16 that are inclined to the left and right around the top thereof. In this state, one blood-sampling tube is held between the left and right inclined surfaces 16 of the convex portion 15 and the corresponding inclined portions 20 of the side walls 11, respectively. Further, when the cam 13 is at center position thereof, each groove 14 faces the corresponding side wall 11 of the casing 10.

The state shown in FIG. 9(a) is an initial state, in which any blood-sampling tube is not contained the grooves 14 and the arms 22a of the blood-sampling tube detection sensors 22a are in an initial position thereof. From this state, as shown in FIG. 9(b), the cam 13 is rotated leftward by a certain amount so that the right groove 14 appears inside the casing 10. Then, when the horizontal distance between the right side wall 11 and the lowermost end of the corresponding inclined surface 16 becomes larger than the diameter of the blood-sampling tube, a blood-sampling tube held in said V-shaped space enters the groove 14 on the right side. At the same time, a blood-sampling tube is held by the inclined portion 20 of the right side wall 11 while being in contact with the blood-sampling tube that has entered the groove 14.

From this state, the cam 13 is rotated to the right, then the cam 13 is returned to the center position thereof while the blood-sampling tube is contained in the right side groove 14 (See FIG 9(c) . In this state, each groove 14 faces the corresponding wall 11 of the casing 10 while the right side groove 14 has held the blood-sampling tube therein. Further, in this state, one blood-sampling tube is held between the left and right inclined surfaces 16 of the convex portion 15 and the corresponding inclined portions 20 of the side walls 11, respectively. Furthermore, in this state, the arm 22a of the right side blood-sampling tube detection sensor 22 is pushed down by the blood-sampling tube contained in the right side groove 14 and the sensor 22b detects the presence of the blood-sampling tube in the right side groove 14.

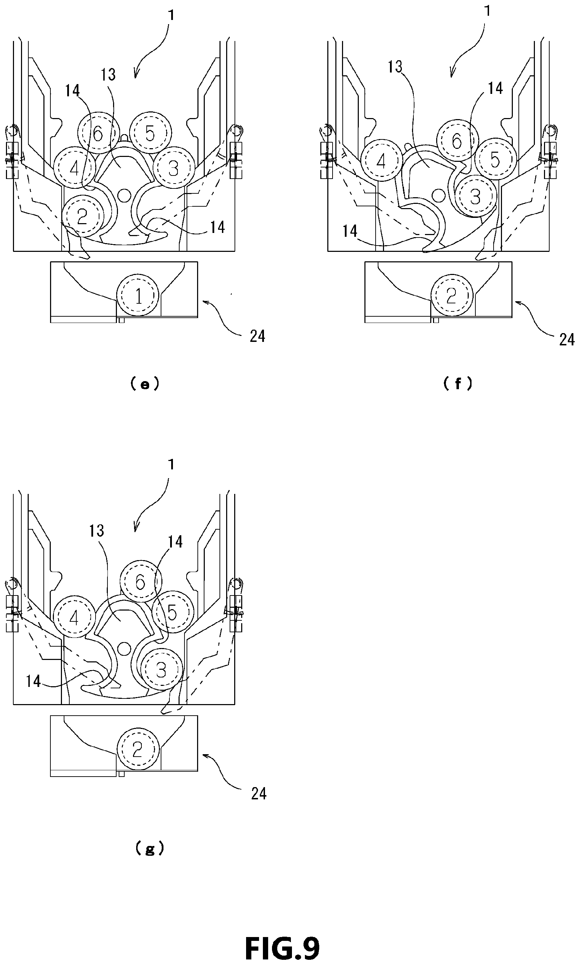

Then, as shown in FIG. 9(d), when the cam 13 is rotated to the right by a certain amount, the right side groove 14 moves from the position facing the right side wall 11 to the lower opening. As a result, the blood-sampling tube contained in the groove 14 is dropped and supplied to the transfer unit 2 arranged below the blood-sampling tube containing unit 1. In the FIG. 9, reference numeral 24 indicates a transfer bucket that constitutes a part of transfer unit 2. The configuration of the transfer unit 2 will be described later. At the same time, the left side groove 14 of the cam 13 appears inside the casing 10 while the horizontal distance between the left side wall 11 and the lowermost end of the corresponding inclined surface 16 becomes larger than the diameter of the blood-sampling tube. As a result, a blood-sampling tube enters the groove 14 on the left side.

Further, from this state, the cam 13 is rotated leftward to the center position thereof so that the blood-sampling tube is held in the left side groove 14 (See FIG. 9(e)). Then, the cam 13 is rotated leftward by a certain amount so that the blood-sampling tube held in the left side groove 14 is dropped and supplied to the transfer unit 2 and a blood-sampling tube is contained in the right side groove 14 (See FIG 9(f)). After that the cam 13 is rotated rightward to its center position so that the blood-sampling tube has been held in the right side groove 14 (FIG. 9(g)).

As described above, every time the cam 13 is rotated leftward and rightward, one blood-sampling tube is contained in one groove 14 and the blood-sampling tube contained in the other groove 14 is dropped and supplied to the transfer unit 2. Therefore, after the cam 13 is operated from the initial state, one blood-sampling tube is always contained in one of the grooves 14 so that the taking out of the blood-sampling tube will be faster.

In addition, every time the cam 13 is rotated leftward and rightward, the protrusion 17 provided on the top of the convex portion 15 moves the blood-sampling tubes contained in the casing 10 upward and aligns them so that the attitudes of the blood-sampling tubes is prevented from tilting in an unexpected direction, thus ensuring the removal of the blood-sampling tube from the casing 10.

Further, since the convex portion 15 has the left and right inclined surfaces 16 around the top thereof, every time the cam 13 is rotated leftward and rightward, the blood-sampling tubes in the blood-sampling tube containing unit 1 are evenly distributed to right and left sides. Therefore, the blood-sampling tubes contained in the blood-sampling tube containing unit 1 can be evenly taken out.

Furthermore, by providing the protrusions 21 on both side wall 11, the plural blood-sampling tubes do not enter the region between each inclined surface 16 of the convex portion 15 and the inclined portion 20 of each side walls 11. Therefore, it is possible to prevent the blood-sampling tube from biting in said area, the problem such as poor rotation of the cam 13 due to biting the blood-sampling tubes do not occur.

Next, the configuration of the transfer unit 2 will be described.

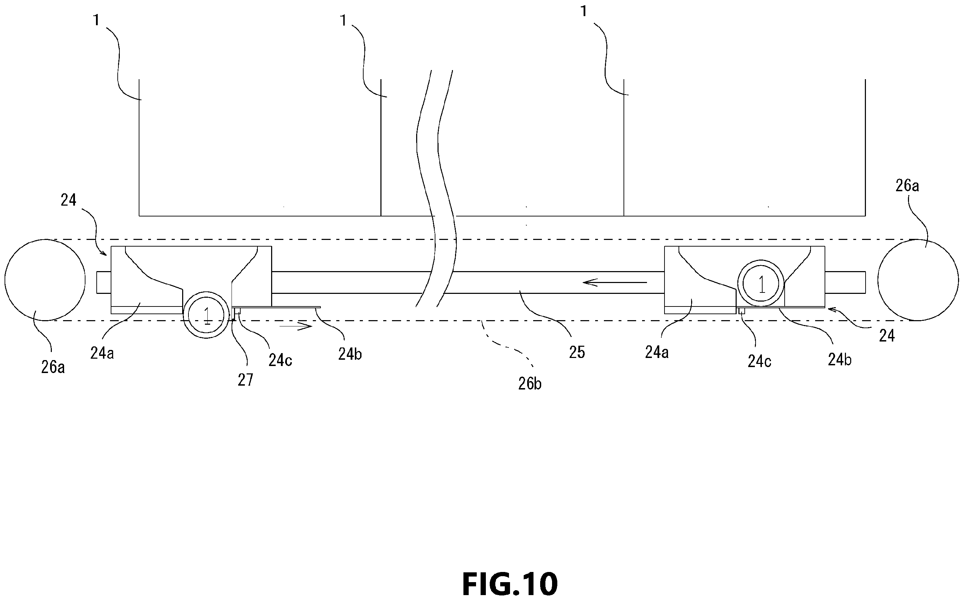

As shown in FIGS. 2 and 10, the transfer unit 2 comprises a pair of guide rails 25 horizontally arranged below the blood-sampling tube containing unit 1. The guide rails 25 are arranged along the blood-sampling tube containing unit 1. And the transfer unit 2 further comprises a pair of pulleys 26a, a belt 26b spanned between the pulleys 26a, and the transfer bucket 24. The transfer bucket 24 is moved reciprocally along the guide rails 25 by the belt 26b.

As shown in FIG. 10, the transfer bucket 24 has a bucket portion 24a whose upper and lower sides are open, and a slide lid member 24b capable of opening and closing the lower side of the bucket portion 24a. Said slide lid member 24b is always urged by an appropriate urging part (not shown) such as a spring in a direction to close the lower side of the bucket portion 24a. The lid member 24b is formed with a protrusion 24c projecting downward. While the bucket portion 24a is moved to a blood-sampling tube supply position, the protrusion 24c is engaged with an engagement 27 arranged at an appropriate position and as a result, the lid member 24b is pushed against the force of said urging part (not shown) in the direction to open the lower side of the bucket portion 24a.

The transfer bucket 24 is moved, with the lid member 24b keeping the lower side of the bucket portion 24a closed, along the guide rails 25 to a position below the blood-sampling tube containing unit 1 in which the blood-sampling tubes corresponding to an order from a doctor are contained.

At that position described above, the blood-sampling tube required to the examination is dropped and supplied to the bucket portion 24a from the cam 13 and then the transfer bucket 24 is moved to the blood-sampling tube supply position shown in the left side in FIG.2 while holding the received blood-sampling tube. Said engagement 27 is fixed at the position corresponding to the blood-sampling tube supply position. While the bucket 24 is moved to a certain position the protrusion 24c of the lid member 24b engages with the engagement 27 so as to lock the lid member 24b. And then the transfer bucket portion 24a is only moved to the blood-sampling tube supply position so that the lid member 24b is opened and therefore the lower side of the bucket portion 24a is opened and the blood-sampling tube contained in the bucket portion 24a is dropped and supplied downward (See FIG. 10).

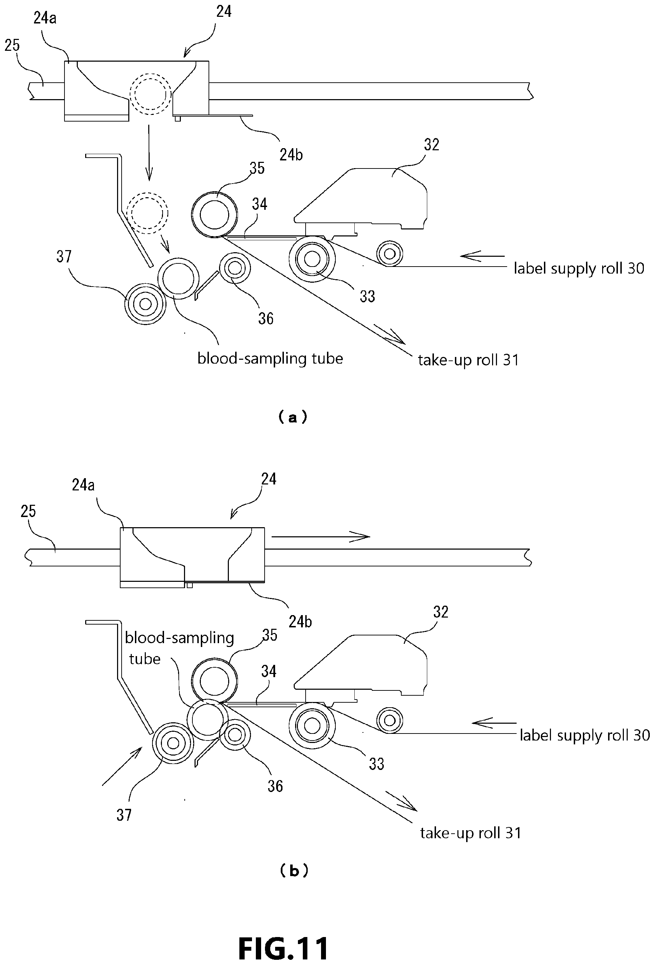

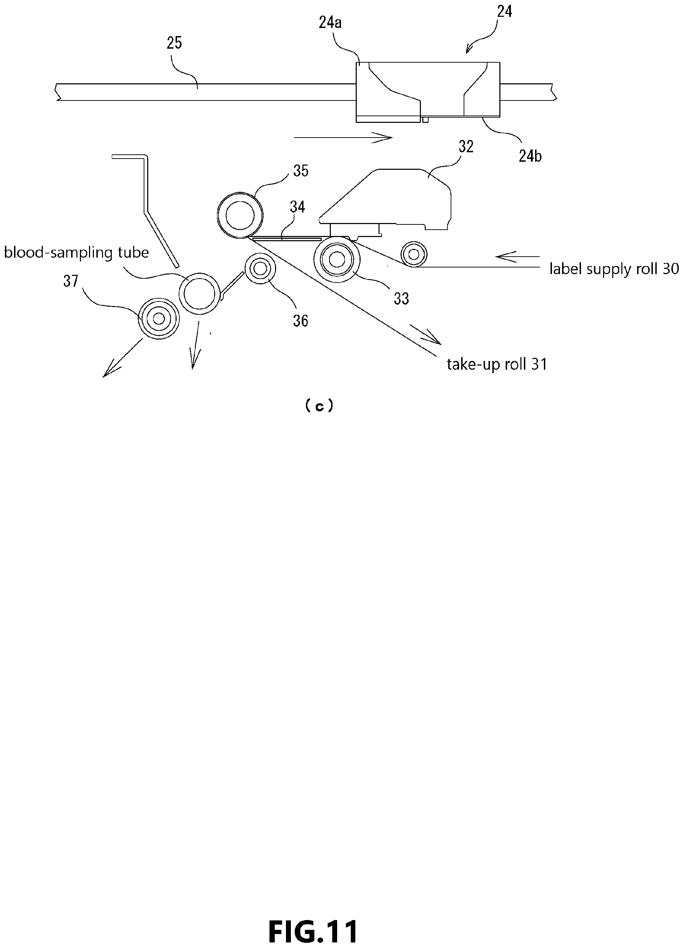

The blood-sampling tube dropped and supplied from the transfer bucket 24 is further dropped and supplied to a label pasting position of the label printing and pasting unit 3. The label printing and pasting unit 3 comprises a label supply roll 30 for supplying a continuous label-attached release mount paper (See FIG.2) and a take-up roll 31 for winding the mount paper (See FIG.2). The label printing and pasting unit 3 further comprises a printing head 32, a platen roller 33 and a release plate 34 as shown in FIG. 11.

When the label-attached release mount paper fed from the label supply roll 30 passes between the printing head 32 and the platen roller 33, information regarding to the examination of a patient is printed on a label of the mount paper by the printing head 32. Said information may includes patient name, patient ID and sample ID or the like. Then the mount paper is bent at an acute angle as it passes through the release plate 34. At this time, only the printed label, which is more elastic and less likely to be bent than the mount paper, is released from the mount paper and goes straight to be pasted to the blood-sampling tube at the label pasting position.

The label painting and pasting unit 3 comprises a label pasting roller 35, an auxiliary roller 36 and the pressure roller 37.

The pressure roller 37 is configured to be movable between three positions. Said three positions are the label pasting position in which the blood-sampling tube is supported by the label pasting roller 35 and the auxiliary roller 36 as well as the pressure roller 37, a blood-sampling tube receiving position in which the pressure roller 37 receives the blood-sampling tube dropped and supplied from the transfer unit2, and a blood-sampling tube discharge position where the pressure roller 37 drops and discharges the label pasted blood-sampling tube onto the tray 5.

FIG. 11(a) shows a state where the pressure roller 35 is at the blood-sampling tube receiving position, FIG. 11(b) shows a state where the pressure roller 35 is at the label pasting position, and FIG11(c) shows a state where the pressure roller 37 is at the blood-sampling tube discharge position. The blood-sampling tube dropped and supplied from the transfer unit 2 is supported by the pressure roller 37 at the blood-sampling tube receiving position (See FIG. 11(a)). Then, the pressure roller 37 is moved to the label pasting position while pushing up the blood-sampling tube. At the label pasting position the blood-sampling tube is supported at three points by the pressure roller 37 in addition to the label pasting roller 35 and the auxiliary roller 36. The printed label released from the mount paper is inserted between a surface of the blood-sampling tube and the label pasting roller 35 at the label pasting position and then the printed label is pasted on the surface of the blood-sampling tube while the blood-sampling tube is rotated by the label pasting roller 35 (See FIG. 11(b)).

After the printed label is completely pasted on the blood-sampling tube, the pressure roller 37 is moved down to the blood-sampling tube discharge position while supporting the label pasted blood-sampling tube (See FIG. 11(c)). As a result, the label pasted blood-sampling tube is dropped and discharged to the tray 5 located below.

The label printer 4 for hand-pasting prints a blood-sampling instruction sheet based on the order from the doctor and discharges it onto the tray 5. In addition, the label printer 4 also prints a label for hand-pasting as necessary and discharges it onto the tray 5.

The automatic blood-sampling tube preparation device has a door 6a configured to be openable and closeable around a longitudinal axis of an upper surface of the housing 6 as shown in FIG.1 so as to allow the blood-sampling tube to be inserted into each blood-sampling tube containing unit 1 from the front side thereof by opening the door 6a.

Further, in FIG.1, reference numeral 8 indicates a touch-screen type monitor provided on the front surface of the automatic blood-sampling tube preparation device. The monitor 8 displays information such as the current operation status of the automatic blood-sampling tube preparation device, the number of remaining blood-sampling tubes in the preparation device, and the remaining amount of the labels or the like. In addition, the monitor 8 may be used as an interface through which a user can input information and signals.

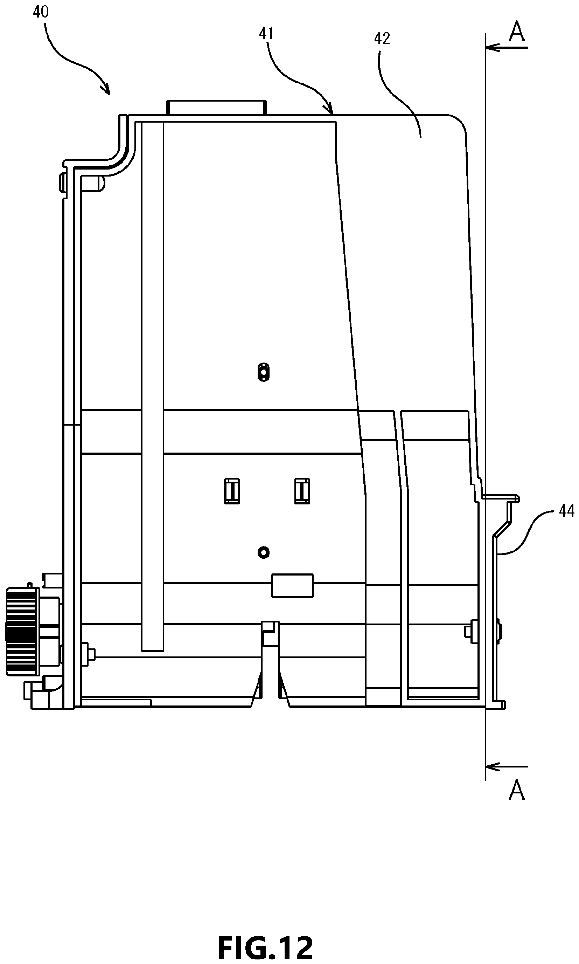

Hereinafter, a second embodiment of blood-sampling tube containing units in an automatic blood-sampling tube preparation device according to the present invention with reference to FIGS 12 to 16.

Among the constituent members of the second embodiment, constituent members equivalent to those of the first embodiment are designated by the same reference numerals as those used in the first embodiment, and detailed description thereof will be omitted.

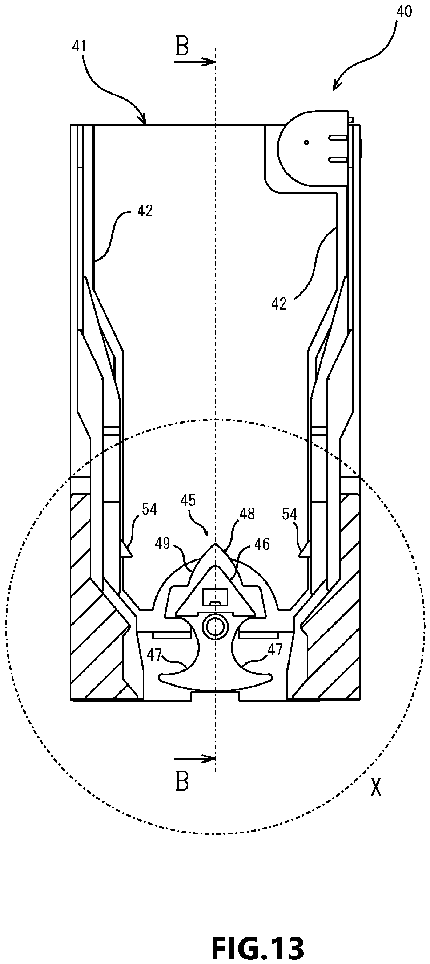

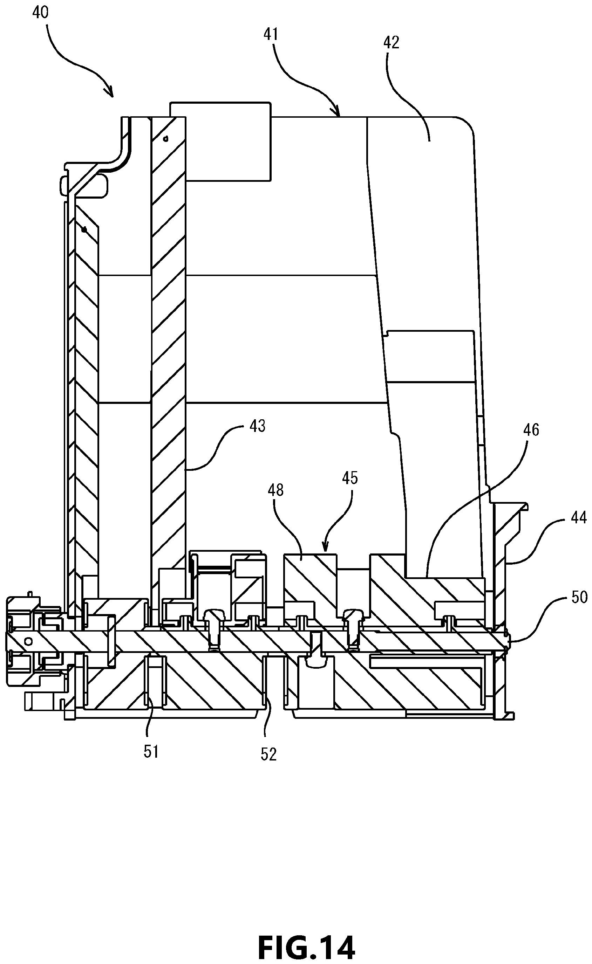

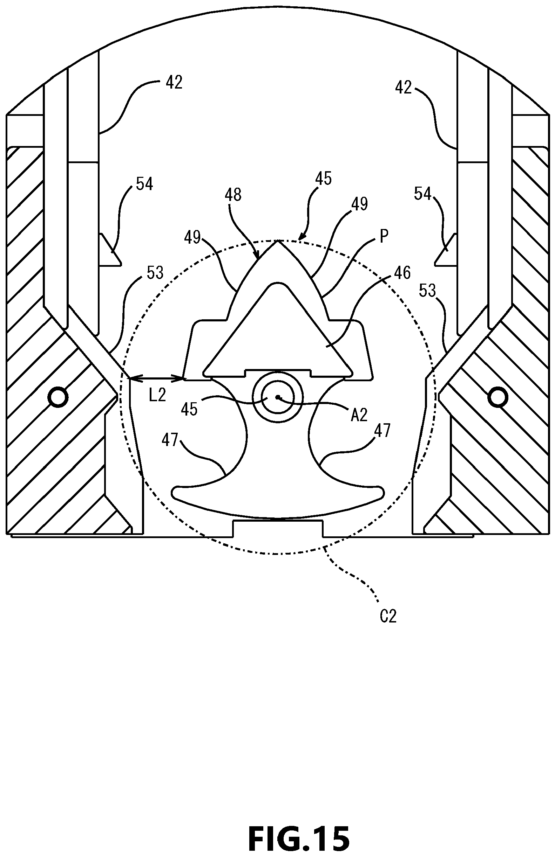

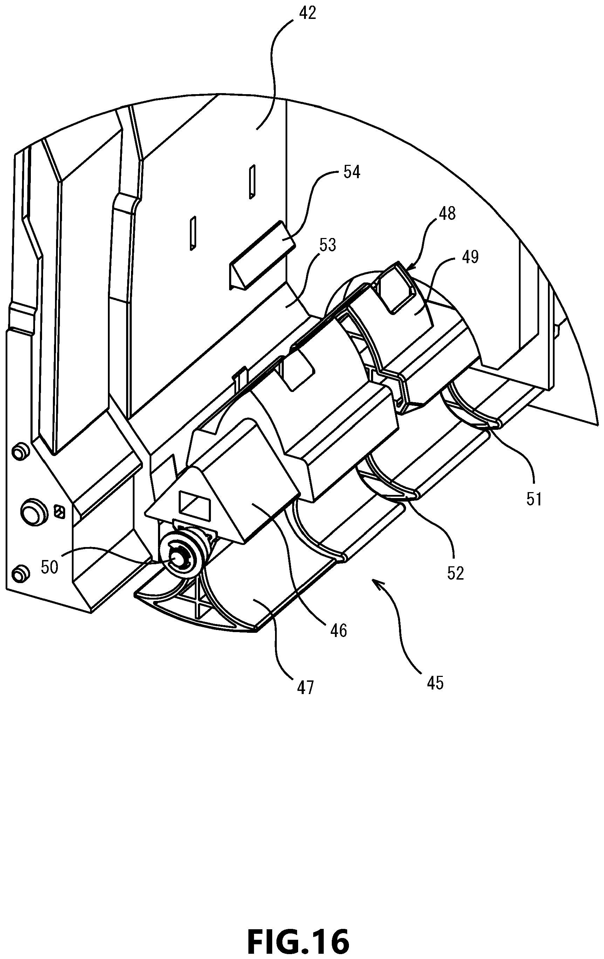

FIG. 12 shows a side view of one of a plurality of blood-sampling tube containing section 40 arranged horizontally. Fig. 13 is a cross-sectional view taken along line A-A in Fig. 12. Fig.14 is a cross-sectional view taken along line B-B in Fig.13. FIG.15 is a schematic enlarged view of a portion of a circle X in FIG. 13. FIG.16 shows a perspective view of a part of a housing and a cam.

As shown in the drawings, each blood-sampling tube containing unit 40 comprises a casing 41. The casing 41 has a pair of left and right side walls 42, a rear wall 43, and a front wall 44. The front wall 44 covers a lower portion of the front surface of the casing 41. A bottom surface, an upper surface, and an upper portion of the front surface of the casing 41 are opened. A blood-sampling tube taking-out cam 45 is provided in the opening portion of the bottom surface of the casing 41.

The blood-sampling tube taking-out cam 45 is configured to take out the blood collection tubes contained in the blood-blood sampling tube containing unit 40 one by one and drop the taken blood-sampling tube into the transfer unit 2 (refer to the transfer unit 2 of the first embodiment) located below.

As shown in FIGS. 13 to 15, the blood-sampling tube taking-out cam 45 has a length extending over the entire length of the casing 40 and is arranged in the widthwise center of the casing 40 so as to close the bottom opening of the casing 40.

Further, as shown in FIGS 14 to 16, the blood-sampling tube taking-out cam 45 has a stepped shape has a reduced diameter at the front portion 46 thereof so that a cap of the blood-sampling tube can be avoided from the cam 45 when the blood-sampling tubes are accommodated in the casing 40.

As shown in FIGS. 13 and 15, the blood-sampling tube taking-out cam 45 has a pair of left and right recessed grooves 47 extending in the longitudinal direction in the lower portion thereof. Each of the grooves 47 is dimensioned to accommodate one blood-sampling tube contained in the blood-sampling tube containing unit 40.

An upper part of the blood-sampling tube taking-out cam 45 is formed with a convex portion 48 which projects inward (that is, upward) of the blood-sampling tube containing unit 40, and the convex portion 48 forms a pair of inclined bottom surfaces (inclined surfaces) 49 that are symmetrical each other when the blood-sampling tube taking-out cam 45 is at the center position thereof. Specifically, as shown in FIG. 15, said convex portion 48 has a stepped shape in its cross-sectional shape. Said stepped shape is a shape in its cross-section that inclines downward from the apex of the convex portion 48 at an acute angle, then extends outward in a substantially horizontal direction, and then further inclines downward at an acute angle. In addition, as shown in FIG. 15, the convex portion 48 is shaped in its cross-sectional shape so that its profile P defining the inclined surfaces 49 thereof is within a true circle C2 having a radius extending from a rotational axis A2 of the blood-sampling tube taking-out cam 45 to an apex of the convex portion 48. As a result, the convex portion 48 forms a relatively acute-angled inclined surfaces 49 centering on the apex of the blood-sampling tube taking-out cam 48 when the cam 48 is at the center position thereof.

As described above, since each inclined surface 49 of the convex portion 48 is formed into the stepped shape, the lower end (that is, the portion opposite to the cap) of the blood-sampling tube located near the cam 45 falls to the stepped portion so that the direction of the blood-sampling tube is aligned with the longitudinal direction of the cam 45. This configuration prevents the attitude of the blood-sampling tube from tilting obliquely with respect to the longitudinal direction of the cam 45 while it is rotated.

Said blood-sampling tube taking-out cam 45 described above is configured to be rotatable left and right about a rotation shaft 50 thereof by a motor or the like (not shown). When the cam 45 is rotated a certain amount in the right direction, the blood-sampling tube enters the groove 47 located on the left side of the cam 45 whereas the blood-sampling tube contained in the groove 47 located on the right side of the cam 45 falls into the transfer unit 2. Conversely, when the cam 45 is rotated a certain amount to the left, the blood-sampling tube enters the groove 47 located on the right side of the cam 45 whereas the blood-sampling tube contained in the groove 47 located on the left side of the ca 45 falls into the transfer unit 2. The amount of the rotation of the cam 45 can be appropriately adjusted according to the size of the blood-sampling tube accommodated in the blood-sampling tube containing unit 40. Specifically, for example, in the case of the blood-sampling tube having a diameter of 12mm, the cam 45 may be rotated 38 degrees, and in the case of the blood-sampling tube having a diameter of 16mm, the cam 45 may be rotated 44 degrees.

Further, as shown in FIG. 14, the cam 45 has an annular notch 51 formed at the rear side in the longitudinal direction thereof. In the embodiment, the blood-sampling tube containing section 40 is configured so that the length of the casing 41 can be adjusted according to the tube length of the blood-sampling tube to be used. When the rear wall 43 for adjusting the length of the casing 41 is attached the notch 51 prevents the rear wall 43 from coming into contact with the cam 45.

Furthermore, the blood-sampling tube containing unit 40 configured as described above comprises the blood-sampling tube detection sensor 22 (not shown) which is configured same as the sensor 22 described in the first embodiment. The cam 45 is formed with an annular notch 52 at the approximate center in the longitudinal direction thereof (See FIG. 14). The notch 52 prevents the blood-sampling tube detection sensor 22 from coming into contact with the cam 45.

On the other hand, as shown in FIG. 15, inclined portions 53 that are inclined obliquely downward in the direction toward the convex portion 48 of the convex portion 48 of the cam are formed on both side walls 42 of the casing 40 same as the first embodiment. Before the cam 45 is rotated and the blood-sampling tube is contained in the groove 47, the blood-sampling tube is held between the inclined portion 53 and the inclined surface 49 formed by the convex portion 48. That is, since the horizontal distance L2 (See FIG. 15) between the lower end of inclined portion 53 and the inclined surface 49 is smaller than the diameter of the blood-sampling tube, blood-sampling tube cannot enter the groove 47. By providing the inclined portions 53 on the side walls 42, a V-shaped space is be formed between each inclined surface 49 of the convex portion 48 and the corresponding inclined portion 53 formed on the side wall 42 especially when the cam 45 is at the center position thereof. Therefore, one blood-sampling tube is guided to the lowermost end of the V-shaped space and held at that position.

Further, protrusions 54 extending in the longitudinal direction of the casing 41 are formed above the inclined portion 53 of the rear parts of both side walls 42 in the longitudinal direction (in particular, see FIGS. 15 and 16). Each protrusion 54 is formed so as to be located at a height substantially corresponding to the top of the convex portion 48 when the cam 45 is at the center position thereof. This configuration prevents a plurality of blood-sampling tubes from entering between the inclined surfaces 49 of the convex portion 48 of the cam 45 and the inclined portions 53 formed on the both side walls 42. As a result, only one blood-sampling tube is held between the inclined surfaces 49 of the convex portion 48 and each of the inclined portions 53 formed on the side walls 42 respectively so that the plural tubes are prevented from entering each space between the inclined surfaces 49 and each of the inclined portions 53 and from causing biting that hinders the rotation of the cam 45.

The width of the casing 41 of the blood-sampling tube containing unit 40 may be set according to the number and the diameter of the blood-sampling tubes to be contained same as the first embodiment.

In the first embodiment, the inclined surfaces 16 formed by the convex portion 15 of the cam 13 is gently curved, and in the second embodiment, the inclined surfaces 49 formed by the convex portion 48 of the cam 45 is formed in the stepped shape. However, the shape of the convex portion of the blood-sampling tube taking-out cam is not limited to 1^{st} and 2^{nd} embodiments and may be formed, for example, so that the inclined surfaces thereof are linear, that is, the cross section of the convex portion may be an isosceles triangle.

Further, in the first embodiment the protrusion 17 is formed on the top of the convex portion 15, however, the protrusion 17 is not an essential element of the present invention. Furthermore, the position at which the protrusion 17 is provided is not limited to the first embodiment, the protrusion 17 may be provided at any position as long as the protrusion 17 can move the blood-sampling tubes contained in the blood-sampling tube containing unit 1 up and down when the blood-sampling tube taking-out cam 13 is rotated. That is, for example, the protrusion 17 may be formed on the inclined surfaces 16 of the convex portion 15, not on the top thereof.

In addition, in the first embodiment, the protrusion 17 provided on the top of the convex portion 15 is configured to continuously extend in the longitudinal direction of the blood-sampling tube taking out cam 13, however, the configuration of the protrusion 17 is not limited to the first embodiment, and for example, the protrusion 17 may be configured to intermittently extend in the longitudinal direction of the cam 13.

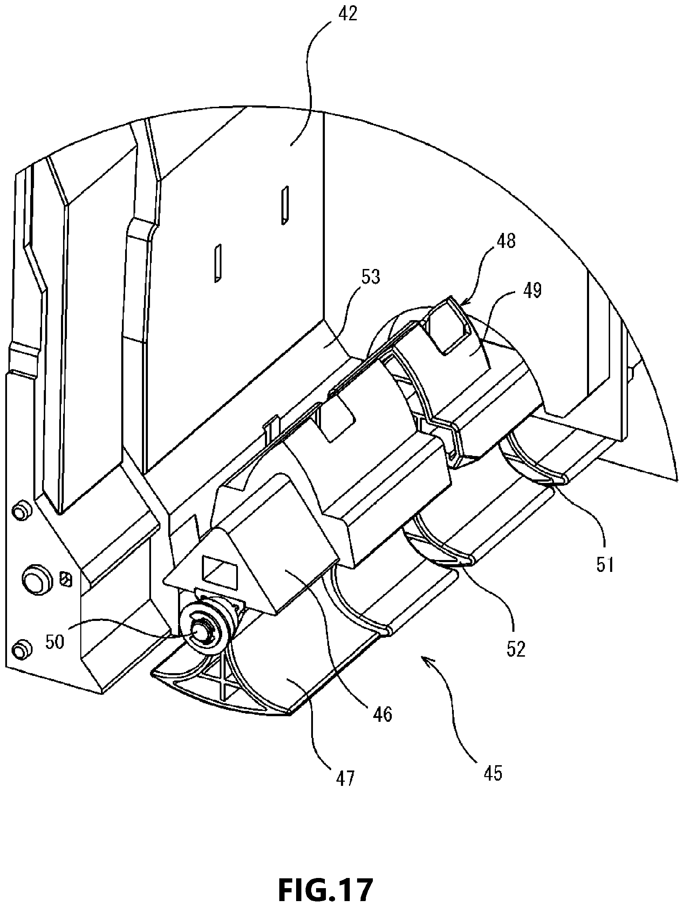

Further, in the first and second embodiment, the protrusions 21; 54 are provided on both side walls 11; 42 of the casing 10; 41, however, the protrusions 21; 54 are not essential element of the present invention, therefore, the protrusions need not be provided on the walls as shown in FIG. 17. FIG. 17 is a perspective view of a third embodiment of the present invention corresponding to FIG. 16, in which there is no protrusion on the walls. The configuration of the third embodiment is the same as that of the second embodiment except that the side walls 42 are not provided with protrusions, and thus the same elements are designated by the same reference numeral and detail description thereof will be omitted.

Furthermore, in the second embodiment, for example, in the case that blood-sampling tubes having a diameter of 12mm are contained in the blood-sampling tube containing unit 1, the blood-sampling tube taking-out cam 45 is controlled so that the cam 45 is rotated from the center position to right or left by 38 degree whereby one blood-sampling tube is entered the groove 47 located on the opposite side of the rotation direction, and then the cam 45 is returned to the center position thereof, and further is rotated by 38 degree in the opposite direction. However, the operation of the cam 45 is not limited to the second embodiment, and for example, the cam 45 may be controlled so that after the cam 45 is rotated 38 degrees from its center position to the right the cam 45 is rotated 25 degrees to the opposite direction, and it is rotated 38 degrees to the right again, and then it is returned to the center position thereof. In the above method, since the operation of the cam 45 is controlled so that the cam 45 is rotated in one direction, then returned to the opposite direction, and further rotated in said one direction, for example, in the case that the cam 45 is rotated in right direction, even if the blood-sampling tube fails to enter the groove 47 in the first right rotation, it will enter the groove 47 in the second right rotation. As a result, it is ensured that the blood-sampling tube is reliably entered the groove 47. Further, by returning the cam 45 once in the opposite direction as described in the above, it is ensured that the cap portion of the blood-sampling tube located on the cam 45 is reliably entered the front portion 46 of the cam 45 having the reduced diameter so that the attitude of the blood-sampling tube is kept horizontal.

### [Description of reference numerals]

- 1: blood-sampling tube containing unit
- 2: transfer unit
- 3: label printing and pasting unit
- 4: label printer for hand pasting
- 5: tray
- 6: housing
- 8: monitor
- 10: casing
- 11: left and right side walls
- 12a: rear wall
- 12b: front wall
- 13: blood-sampling tube taking out cam
- 14: groove
- 15: convex portion
- 16: bottom wall (inclined surface)
- 17: protrusion
- 18: rotation shaft
- 19: annular notch
- 20: inclined portion
- 21: protrusion
- 22: blood-sampling tube detection sensor
- 22a: arm
- 22b: sensor
- 23: cam origin position detection sensor
- 23a: shield plate
- 23b: sensor
- 24: transfer bucket
- 24a: bucket portion
- 24b: slide lid member
- 24c: protrusion
- 25: guide rail
- 26a: pulley
- 26b: belt
- 27: engagement
- 28: bracket
- 29: bracket
- 30: label supply roll
- 31: take-up roll
- 32: printing head
- 33: platen roller
- 34: release plate
- 35: label pasting roller
- 36: auxiliary roller
- 37: pressure roller
- 40: blood-sampling tube containing unit
- 41: casing
- 42: left and side walls
- 43: rear wall
- 44: front wall
- 45: blood-sampling tube taking-out cam
- 46: front portion
- 47: groove
- 48: convex portion
- 49: bottom wall (inclined surface)
- 50: rotation shaft
- 51: notch
- 52: notch
- 53: inclined portion
- 54: protrusion
- 60: blood-sampling tube containing unit
- 61: groove
- 62: roller
- 63: conveyer
- 64: bucket

## Claims

1. An automatic blood-sampling tube preparation device comprising
a plurality of blood-sampling tube containing units (1; 40) for containing the blood-sampling tubes, which are arranged in a horizontal direction,
a transfer unit (2) arranged under the blood-sampling tube containing units (1; 40) and along them,
a label printing and pasting unit (3) for printing information about a patient on a label and pasting the printed label on a tube, which is arranged under the transfer unit (2), and
a blood-sampling tube collection unit (5) for collecting label pasted tubes, which is arranged under the label printing and pasting unit (3),
wherein
each blood-sampling tube containing unit (1; 40) comprises a casing (10; 41) with an open bottom, capable of stacking and containing a plurality of blood-sampling tubes in a horizontal state in a direction orthogonal to the direction in which the blood-sampling tube containing units (1; 40) are arranged in parallel,
a blood-sampling tube taking-out cam (13; 45) is arranged at the center of the open bottom of each casing (10; 41), which is configured to close the open bottom of the casing (10; 41), to take-out the blood-sampling tube contained in the casing (10; 41) one by one, and to transfer the taken out tube to the transfer unit (2) arranged below the casing,
said blood-sampling tube taking-out cam (13; 45) is configured to extend along a longitudinal direction of the blood-sampling tube containing unit (1; 40) and to be rotatable left and right about a longitudinal axis thereof,
a convex portion (15; 48) is formed on an upper portion of the blood-sampling tube taking-out cam (13; 45), in which said convex portion (15; 48) has a mountain-shaped cross section protruding upward, and forms symmetrical inclined surfaces (16; 49) extending downwardly from a top thereof to side walls (11; 42) of the blood-sampling tube containing unit (1; 40), said inclined surfaces (16, 49) extending along the longitudinal direction of the convex portion (15; 48),
a pair of left and right grooves (14; 47) extending along the longitudinal direction of the blood-sampling tube taking-out cam (13; 45) at the lower part thereof,
said taking-out cam (13; 45) being configured to direct said convex portion (15; 48) to the inside of the blood-sampling tube containing unit (1; 40) to form the bottom surface of the blood-sampling tube containing unit (1; 40) and to direct each groove (14; 47) thereof to the corresponding side wall (11; 42) of the blood-sampling tube containing unit (1; 40), when the taking-out cam (13; 45) is at its center position,
wherein
when the blood-sampling tube taking-out cam (13; 45) is rotated in one direction form its center position, one of grooves (14; 47) appears inside the blood-sampling tube containing unit (1; 40) so that one of blood-sampling tubes contained in the blood-sampling tube containing unit (1; 40) enters one groove (14; 47) while the blood-sampling tube contained in other groove (14; 47) is dropped and supplied to the transfer unit (2),
and when the blood-sampling tube taking-out cam (13; 45) is rotated in other direction form its center position, said other groove (14; 47) appears inside the blood-sampling tube containing unit (1; 40) so that one of blood-sampling tubes contained in the blood-sampling tube containing unit (1; 40) enters said other groove (14; 47) while the blood-sampling tube contained in said one groove (14; 47) is dropped and supplied to the transfer unit (2).

2. The automatic blood-sampling tube preparation device according to claim 1, **characterized in that** inclined portions (20; 53) are provided at portions of both side walls (11; 42) of the blood-sampling tube containing unit (1; 40), corresponding to the inclined surfaces (16; 49) of the convex portion (15; 48), each of the inclined portions (20; 53) is inclined obliquely downward toward the lower end of each inclined surface (16; 49).

3. The automatic blood-sampling tube preparation device according to claims 1 or 2, **characterized in that** the convex portion (15; 48) of the blood-sampling tube taking-out cam (13; 45) is shaped in its cross section so that its profile defining the inclined surfaces (16; 49) is within a true circle (C; C2) having a radius extending from a rotational axis (A; A2) of the blood-sampling tube taking-out cam (13; 45) to the top of the convex portion (15; 48).

4. The automatic blood-sampling tube preparation device according to any one of claim 1 to 3, **characterized in that** one end portion in the longitudinal direction of the blood-sampling tube taking-out cam (13; 45) is formed in a stepped shape so as to avoid a cap portion of the blood-sampling tube from interfering with the blood-sampling tube taking-out cam (13; 45).

5. The automatic blood-sampling tube preparation device according to any one of claims 1 to 4, **characterized in that** the blood-sampling tube containing unit (1; 40) is configured to be capable adjusting its length in the longitudinal direction according to the length of the blood-sampling tube to be contained.

6. The automatic blood-sampling tube preparation device according to any one of claims 1 to 5, **characterized in that** the blood-sampling tube containing unit (1; 40) is configured to be capable adjusting its width according to the number of the blood-sampling tubes to be contained.

7. The automatic blood-sampling tube preparation device according to any one of claim 1 to 6, **characterized in that** a continuous protrusion (17) extending in the longitudinal direction or a plurality of protrusions intermittently arranged along the longitudinal direction are formed on the top of the convex portion (15) of the blood-sampling tube taking-out cam (13).

8. The automatic blood-sampling tube preparation device according to any one of claim 1 to 6, **characterized in that** a continuous protrusion extending in the longitudinal direction or a plurality of protrusions intermittently arranged along the longitudinal direction are formed on the inclined surfaces (16; 49) formed by the convex portion (15; 48) of the blood-sampling tube taking-out cam (13; 45).

9. The automatic blood-sampling tube preparation device according to any one of claim 1 to 6, **characterized in that** each of the inclined surfaces (49) formed on the convex portion (48) of the blood-sampling tube taking-out cam (45) has a stepped shape.

## Patentansprüche

1. Automatische Blutprobenröhrchen-Präparationsvorrichtung mit einer Anzahl von Blutprobenröhrchen-Aufnahmeeinheiten (1; 40) zur Aufnahme der Blutprobenröhrchen, die in einer horizontalen Richtung angeordnet sind,
einer Übertragungseinheit (2), die unter den Blutprobenröhrchen-Aufnahmeeinheiten (1; 40) und entlang dieser angeordnet ist,
einer Etiketten-Druck- und Klebeeinheit (3) zum Drucken von Informationen bezüglich eines Patienten auf einem Etikett und zum Kleben des bedruckten Etiketts auf ein Röhrchen, die unter der Übertragungseinheit (2) angeordnet ist, und
einer Blutprobenröhrchen-Sammeleinheit (5) zum Sammeln von etikettierten Röhrchen, die unter der Etiketten-Druck-und Klebeeinheit (3) angeordnet ist,
wobei jede Blutprobenröhrchen-Aufnahmeeinheit (1; 40) ein Gehäuse (10; 41) mit einem offenen Boden aufweist, das in der Lage ist, eine Anzahl von Blutprobenröhrchen in einem horizontalen Zustand und in einer Richtung orthogonal zu der Richtung aufzunehmen, in der die Blutprobenröhrchen-Aufnahmeeinheiten (1; 40) parallel angeordnet sind,
ein Blutprobenröhrchen-Herausnahmenocken (13; 15) in der Mitte des offenen Bodens jedes Gehäuses (10; 41) angeordnet ist, der ausgebildet ist, um den offenen Boden des Gehäuses (10; 41) zu schließen und das Blutprobenröhrchen, das in dem Gehäuse (10; 41) aufgenommen ist nacheinander herauszunehmen und das herausgenommene Röhrchen zu der Übertragungseinheit (2) zu übertragen, die unterhalb des Gehäuses angeordnet ist,
wobei der Blutprobenröhrchen-Herausnahmenocken (13; 45) ausgebildet ist, um sich entlang einer Längsrichtung der Blutprobenröhrchen-Aufnahmeeinheit (1; 40) zu erstrecken und sich um ihre Längsachse nach rechts und nach links drehbar zu sein,
ein konvexer Teil (15; 48) an einem oberen Teil des Blutprobenröhrchen-Herausnahmenockens (13; 45) ausgebildet ist, wobei der konvexe Teil (15; 48) einen bergförmigen Querschnitt aufweist, der nach oben vorsteht und symmetrische geneigte Flächen (16; 49) bildet, die sich nach unten von seinem Oberen zu Seitenwänden (41; 42) der Blutprobenröhrchen-Aufnahmeeinheit (1; 40) erstrecken, wobei sich die geneigten Flächen (16, 49) entlang der Längsrichtung des konvexen Teils (15; 48) erstrecken,
mit einem Paar von linken und rechten Nuten (14; 47), die sich entlang der Längsrichtung des Blutprobenröhrchen-Herausnahmenockens (13; 45) an seinem unteren Teil erstrecken,
wobei der Herausnahmenocken (13; 45) ausgebildet ist, um den konvexen Teil (15; 48) zur Innenseite der Blutprobenröhrchen-Aufnahmeeinheit (1; 40) zu richten, um die Bodenfläche der Blutprobenröhrchen-Aufnahmeeinheit (1; 40) zu bilden und jeden Nut (14; 47) davon zu der entsprechenden Seitenwand (11; 42) der Blutprobenröhrchen-Aufnahmeeinheit (1; 40) zu richten, wenn der Herausnahmenocken (13; 45) in seiner mitten Position ist, wobei
wenn der Blutprobenröhrchen-Herausnahmenocken (13; 45) in einer Richtung aus seiner mittleren Position gedreht wird, eine der Nuten (14; 47) innerhalb der Blutprobenröhrchen-Aufnahmeeinheit (1; 40) erscheint, so dass eines der Blutprobenröhrchen, das in der Blutprobenröhrchen-Aufnahmeeinheit (1; 40) enthalten ist, eine der Nuten (14; 47) besetzt, während das Blutprobenröhrchen, das in der anderen Nut (14; 47) enthalten ist, fallengelassen wird und an die Übertragungseinheit (2) geliefert wird.

2. Automatische Blutprobenröhrchen-Präparationsvorrichtung nach Anspruch 1, **dadurch gekennzeichnet, dass** geneigte Teile (20; 53) an Abschnitten von beiden Seitenwänden (11; 42) der Blutprobenröhrchen-Aufnahmeeinheit (1; 40) entsprechend den geneigten Flächen (16; 49) des konvexen Teils (15; 18) vorgesehen sind, wobei jeder geneigte Teil (20; 53) schräg nach unten zu dem unteren Ende jeder geneigten Fläche (16; 49) geneigt ist.

3. Automatische Blutprobenröhrchen-Präparationsvorrichtung nach Ansprüchen 1 oder 2, **dadurch gekennzeichnet, dass** der konvexe Teil (15; 48) des Blutprobenröhrchen-Herausnahmenockens (13; 45) in seinem Querschnitt so geformt ist, dass sein Profil, das die geneigten Flächen (16; 49) definiert, innerhalb eines wahren Kreises (C; C2) ist, der einen Radius aufweist, der sich von einer Drehachse (A; A2) des Blutprobenröhrchen-Herausnahmenockens (13; 45) zu dem Oberteil des konvexen Teils (15; 48) erstreckt.

4. Automatische Blutprobenröhrchen-Präparationsvorrichtung nach einem der Ansprüche 1 bis 3, **dadurch gekennzeichnet, dass** ein Endteil in der Längsrichtung des Blutprobenröhrchen-Herausnahmenockens (13; 45) in einer abgestuften Form ausgebildet ist, um so zu verhindern, dass ein Kappenteil des Blutprobenröhrchens mit dem Blutprobenröhrchen-Herausnahmenocken (13; 45) kollidiert.

5. Automatische Blutprobenröhrchen-Präparationsvorrichtung nach einem der Ansprüche 1 bis 4, **dadurch gekennzeichnet, dass** die Blutprobenröhrchen-Aufnahmeeinheit (1; 40) ausgebildet ist, um in der Lage zu sein, ihre Länge in der Längsrichtung entsprechend der Länge des aufzunehmenden Blutprobenröhrchens einzustellen.

6. Automatische Blutprobenröhrchen-Präparationsvorrichtung nach einem der Ansprüche 1 bis 5, **dadurch gekennzeichnet, dass** die Blutprobenröhrchen-Aufnahmeeinheit (1; 40) so ausgebildet ist, dass sie ihre Breite entsprechend der Anzahl der zu enthaltenen Blutprobenröhrchen einstellen kann.

7. Automatische Blutprobenröhrchen-Präparationsvorrichtung nach einem der Ansprüche 1 bis 6, **dadurch gekennzeichnet, dass** ein kontinuierlicher Vorsprung (17), der sich in der Längsrichtung erstreckt, oder einer Anzahl von Vorsprüngen, die abwechselnd entlang der Längsrichtung angeordnet sind, an der Oberseite des konvexen Teils (15) des Blutprobenröhrchen-Herausnahmenockens (13) ausgebildet sind.

8. Automatische Blutprobenröhrchen-Präparationsvorrichtung nach einem der Ansprüche 1 bis 6, **dadurch gekennzeichnet dass** ein kontinuierlicher Vorsprung, der sich in der Längsrichtung erstreckt, oder einer Anzahl von Vorsprüngen, die abwechselnd entlang der Längsrichtung ausgebildet sind, in den geneigten Flächen (16; 49) gebildet sind, die durch den konvexen Teil (15; 48) des Blutprobenröhrchen-Herausnahmenockens (13; 45) gebildet sind.

9. Automatische Blutprobenröhrchen-Präparationsvorrichtung nach einem der Ansprüche 1 bis 6, **dadurch gekennzeichnet, dass** jede der geneigten Flächen (49), die durch die konvexen Teile (48) des Blutprobenröhrchen-Herausnahmenockens (45) gebildet werden, eine gestufte Form hat.

## Revendications

1. Un dispositif automatique de préparation de tubes de prélèvement de sang comprenant
une pluralité d'unités pour contenir des tubes de prélèvement de sang (1 ; 40) pour contenir les tubes de prélèvement de sang, lesquels sont agencées dans une direction transversale,
une unité de transfert (2) agencée sous les unités pour contenir des tubes de prélèvement (1 ; 40) et le long de ces dernières,
une unité d'impression et de collage d'étiquettes (3) pour imprimer des informations concernant un patient sur une étiquette et coller l'étiquette imprimée sur un tube, laquelle unité est agencée sous l'unité de transfert (2), et
une unité de collecte de tubes de prélèvement de sang (5) pour collecter des tubes sur lesquelles des étiquettes ont été collées, laquelle unité est agencée sous l'unité d'impression et de collage d'étiquette (3),
dans lequel
chaque unité pour contenir des tubes de prélèvement de sang (1 ; 40) comprend un boîtier (10 ; 41) avec un fond ouvert, capable de contenir un empilement d'une pluralité de tubes de prélèvement de sang à l'état horizontal dans une direction perpendiculaire à la direction dans laquelle les unités pour contenir des tubes de prélèvement de sang (1 ; 40) sont agencées en parallèle,
une came d'extraction de tubes de prélèvement de sang (13 ; 45) est agencée au centre du fond ouvert de chaque boîtier (10 ; 41) et est configurée pour fermer les fonds ouverts des boîtiers (10 ; 41), pour extraire les tubes de prélèvement de sang contenus dans les boîtiers (10 ; 41) un par un, et transférer les tubes extraits à l'unité de transfert (2) agencée sous les boîtiers,
ladite came d'extraction de tubes de prélèvement de sang (13 ; 45) est configurée pour s'étendre le long d'une direction longitudinale de l'unité pour contenir des tubes de prélèvement de sang (1 ; 40) et pour pouvoir tourner vers la gauche et la droite autour un axe longitudinal de l'unité,
une partie convexe (15 ; 48) est formée sur une partie supérieure de la came d'extraction de tubes de prélèvement de sang (13 ; 45), ladite partie convexe (15 ; 48) ayant une section à section transversale en forme de cône faisant saillie vers le haut et formant des surfaces inclinées symétriques (16 ; 49) s'étendant vers le bas depuis un sommet de celui-ci vers des parois latérales (11 ; 42) de l'unité pour contenir des tubes de prélèvement de sang (1 ; 40), lesdites surfaces inclinées (16, 49) s'étendant le long de la direction longitudinale de la partie convexe (15 ; 48),
une paire de rainures gauche et droite (14 ; 47) s'étendant le long de la direction longitudinale de la came d'extraction de tubes de prélèvement de sang (13 ; 45) à la partie inférieure de celle-ci,
ladite came d'extraction (13 ; 45) étant configurée pour diriger ladite partie convexe (15 ; 48) vers l'intérieur de l'unité pour contenir des tubes de prélèvement de sang (1 ; 40) pour former la surface inférieure de l'unité pour contenir des tubes de prélèvement de sang (1 ; 40) et pour diriger chaque rainure (14 ; 47) de la came vers la paroi latérale correspondante (11 ; 42) de l'unité pour contenir des tubes de prélèvement de sang (1 ; 40), lorsque la came d'extraction (13 ; 45) est à sa position centrale,
dans lequel
lorsque la came d'extraction de tubes de prélèvement (13 ; 45) est tournée dans une direction depuis sa position centrale, l'une des rainures (14, 47) apparaît à l'intérieur de l'unité pour contenir des tubes de prélèvement de sang (1 ; 40) de sorte que l'un des tubes de prélèvement de sang contenus dans l'unité pour contenir des tubes de prélèvement de sang (1 ; 40) pénètre dans une rainure (14 ; 47) pendant que le tube de prélèvement de sang contenu dans une autre rainure (14 ; 47) est laissé tomber et fourni à l'unité de transfert (2),
et lorsque la came d'extraction de tubes de prélèvement de sang (13 ; 45) est tournée dans l'autre direction depuis sa position centrale, ladite autre rainure (14 ; 47) apparaît à l'intérieur de l'unité pour contenir des tubes de prélèvement de sang (1 ; 40) de sorte que l'un des tubes de prélèvement de sang contenus dans l'unité pour contenir des tubes de prélèvement de sang (1 ; 40) pénètre dans l'autre rainure (14 ; 47) pendant que le tube de prélèvement de sang contenu dans la rainure (14, 47) est laissé tomber et fourni à l'unité de transfert (2).

2. Le dispositif automatique de préparation de tubes de prélèvement de sang selon la revendication 1, **caractérisé en ce que** les parties inclinées (20 ; 53) sont prévues à des parties des deux parois latérales (11 ; 42) de l'unité pour contenir des tubes de prélèvement de sang (1 ; 40) correspondant aux surfaces inclinées (16 ; 49) de la partie convexe (15 ; 48), chacune des parties inclinées (20 ; 53) étant inclinée de façon oblique vers le bas vers l'extrémité inférieure de chaque surface inclinée (16 ; 49).

3. Le dispositif automatique de préparation de tubes de prélèvement de sang selon les revendications 1 ou 2, **caractérisé en ce que** la partie convexe (15 ; 48) de la came d'extraction de tubes de prélèvement (13 ; 45) a une section transversale dont la forme est telle que son profil définissant les surfaces inclinées (16 ; 49) est à l'intérieur d'un vrai cercle (C ; C2) ayant un rayon s'étendant d'un axe de rotation (A ; A2) de la came d'extraction de tubes de prélèvement de sang (13 ; 45) jusqu'au sommet de la partie convexe (15 ; 48).

4. Le dispositif automatique de préparation de tubes de prélèvement de sang selon l'une quelconque des revendications 1 à 3, **caractérisé en ce qu'**une partie d'extrémité dans la direction longitudinale de la came d'extraction de tubes de prélèvement de sang (13 ; 45) a une forme étagée de façon à empêcher une partie de sommet du tube de prélèvement de sang d'interférer avec la came d'extraction de tubes de prélèvement de sang (13 ; 45).

5. Le dispositif automatique de préparation de tubes de prélèvement de sang selon l'une quelconque des revendications 1 à 4, **caractérisé en ce que** l'unité pour contenir des tubes de prélèvement de sang (1 ; 40) est configurée pour être capable de régler sa longueur dans la direction longitudinale en fonction de la longueur du tube de prélèvement de sang à contenir.

6. Le dispositif automatique de préparation de tubes de prélèvement de sang selon l'une quelconque des revendications 1 à 5, **caractérisé en ce que** l'unité pour contenir des tubes de prélèvement de sang (1 ; 40) est configurée pour être capable de régler sa largeur en fonction du nombre de tubes de prélèvement de sang à contenir.

7. Le dispositif automatique de préparation de tubes de prélèvement de sang selon l'une quelconque des revendications 1 à 6, **caractérisé en ce qu'**une protubérance continue (17) s'étendant dans la direction longitudinale ou une pluralité de protubérances agencées de façon intermittente le long de la direction longitudinale sont formées au sommet de la partie convexe (15) de la came d'extraction de tubes de prélèvement de sang (13).

8. Le dispositif automatique de préparation de tubes de prélèvement de sang selon l'une quelconque des revendications 1 à 6, caractérisé en qu'une protubérance continue s'étendant dans la direction longitudinale ou une pluralité de protubérances agencées de façon intermittente le long de la direction longitudinale sont formées sur les surfaces inclinées (16 ; 49) formées par la partie convexe (15 ; 48) de la came d'extraction de tubes de prélèvement de sang (13 ; 45).

9. Le dispositif automatique de préparation de tubes de prélèvement de sang selon l'une quelconque des revendications 1 à 6, **caractérisé en ce que** chacune de surfaces inclinées (49) formées sur la partie convexe (48) de la came d'extraction de tubes de prélèvement de sang (45) a une forme étagée.
